(19) **Europäisches Patentamt**
**European Patent Office**
**Office européen des brevets**

(11) **EP 3 599 986 B1**

(12) **FASCICULE DE BREVET EUROPEEN**

(45) Date de publication et mention
de la délivrance du brevet:
**26.05.2021 Bulletin 2021/21**

(21) Numéro de dépôt: **18717533.6**

(22) Date de dépôt: **29.03.2018**

(51) Int Cl.:
***A61B 3/06*** *(2006.01)*

(86) Numéro de dépôt international:
**PCT/EP2018/058120**

(87) Numéro de publication internationale:
**WO 2018/178258 (04.10.2018 Gazette 2018/40)**

(54) **DETERMINATION D'INFORMATIONS D'ISO-LUMINANCE CHROMATIQUE POUR CONTROLER DE FACON PERSONNALISEE UN PROCESSUS INFORMATIQUE**

BESTIMMUNG VON CHROMATISCHEN ISOLUMINANZINFORMATIONEN ZUR PERSONALISIERTEN STEUERUNG EINES COMPUTERPROZESSES

DETERMINATION OF CHROMATIC ISOLUMINANCE INFORMATION FOR CONTROLLING A COMPUTER PROCESS IN A PERSONALISED MANNER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.03.2017 FR 1752814**

(43) Date de publication de la demande:
**05.02.2020 Bulletin 2020/06**

(73) Titulaires:
• **Institut du Cerveau et de la Moelle Epiniere (ICM)**
**75013 Paris (FR)**
• **Centre National de la Recherche Scientifique (CNRS)**
**75016 Paris (FR)**
• **Institut National de la Santé et de la Recherche Médicale (INSERM)**
**75013 Paris (FR)**
• **Assistance Publique-Hôpitaux de Paris (AP-HP)**
**75004 Paris (FR)**
• **Sorbonne Université**
**75006 Paris 6 (FR)**

(72) Inventeurs:
• **POUGET, Pierre**
**75015 Paris (FR)**
• **GÉNIN, Alexis**
**60340 St Leu D'Esserent (FR)**

(74) Mandataire: **Osha Liang**
**2, rue de la Paix**
**75002 Paris (FR)**

(56) Documents cités:
**EP-A1- 1 219 243      US-A- 5 141 305**

Printed by Jouve, 75001 PARIS (FR)

**Description**

DOMAINE DE L'INVENTION

La présente invention concerne l'évaluation de la perception des couleurs chez les mammifères, et plus particulièrement un procédé et un système de génération d'un indicateur ou biomarqueur de la perception des couleurs chez un sujet mammifère, tel qu'un être humain ou un animal. Elle concerne en outre l'utilisation d'un tel indicateur de type iso-luminance chromatique pour le contrôle personnalisé de processus dans un système informatique.

CONTEXTE DE L'INVENTION

La perception des couleurs est fortement variable d'un sujet mammifère à l'autre.
Cette variabilité s'explique traditionnellement par des concentrations, des répartitions et des sensibilités différentes, entre sujets, des photorécepteurs chromatiques (cônes) dans les zones sollicitées de la rétine, généralement sur et autour de la fovéa.
La perception des couleurs évolue avec l'âge du sujet.
Elle s'altère aussi par un dysfonctionnement lié à une pathologie génétique ou acquise de l'œil (les photorécepteurs chromatiques), des voies optiques ou des aires cérébrales visuelles.
L'évaluation de la perception des couleurs est utile pour de nombreuses applications, médicales et non-médicales.
On connaît par exemple le document US 5 141 305 qui décrit l'évaluation d'une activité neuronale de type phase pure en mesurant la réponse pupillaire à un stimulus iso-luminant d'un motif de type points ou barres verticales dont l'unique couleur affichée est alternée entre le vert et le rouge.
Une méthode d'évaluation, dite d'iso-luminance chromatique, consiste à déterminer lorsqu'une combinaison de couleurs, dont l'une varie dans le temps, est perçue par le sujet comme ayant toutes (généralement deux) la même luminance. Cette évaluation est largement utilisée dans les études psychophysiques et neurophysiologiques du traitement visuel chez les mammifères. Elle permet notamment d'évaluer l'intégrité de la perception des couleurs chez le sujet, mais également d'isoler la contribution relative des cellules sensibles à la luminance dans la perception des couleurs.
Longtemps l'évaluation de l'iso-luminance chromatique est restée subjective car se reposant sur des jugements perceptifs des sujets.
Pour permettre une évaluation objective chez des sujets mammifères non verbaux, tels que les animaux et les bébés, des indicateurs ou biomarqueurs de la perception des couleurs chez un sujet mammifère ont été créés. Ces indicateurs ou biomarqueurs sont en quelque sorte des signatures neurologiques et physiologiques objectives de la perception des couleurs testées.
La publication « Screening for color blindness using optokinetic nystagmus » (Cavanagh P. et al., 1984) décrit par exemple la génération d'un signal représentatif du nystagmus de l'œil du sujet en réponse à un scintillement illusoire dans une grille formée de barres rouges et vertes, l'une des couleurs étant graduellement modifiée dans le temps. Ce signal de réponse est un indicateur de la perception de couleurs chez le sujet testé. Notamment l'iso-luminance entre les deux couleurs testées (rouge et verte) est obtenue au moment où la direction de mouvement de l'œil s'inverse dans le signal du nystagmus.
La publication « A new technique for estimating chromatic isoluminance in humans and monkeys » (Chaudhuri A. et al., 1990) divulgue également la génération d'un signal de réponse opto-cinétique à l'affichage d'un stimulus bicolore dynamique. Un changement de direction du nystagmus est également utilisé pour identifier l'iso-luminance entre les deux couleurs (vert et gris) utilisées.
Ces techniques basées sur la réponse opto-cinétique des sujets comme indicateur de la perception des couleurs requièrent cependant une analyse très précise du mouvement des yeux. En effet, les oscillations involontaires des yeux en réponse au stimulus bicolore dynamique sont généralement brutales et rapides. Des dispositifs d'acquisition d'images sophistiqués (par exemple des caméras 100 images/seconde) et des moyens de traitement performants, notamment pour des traitements temps-réel, sont donc nécessaires.
En outre, pour garantir cette précision, il est nécessaire que le regard du sujet ne bouge pas. Cette contrainte est difficile à tenir pour certains sujets, tels que les animaux et les bébés.
De plus, l'utilisation d'un oculomètre pour la mesure des mouvements oculaires nécessite une procédure de calibration de l'oculomètre. Cette procédure de calibration nécessite aussi une réponse de la part des sujets, rendant ces mesures difficiles à faire précisément sur des sujets non-coopérants.
Il existe ainsi un besoin de disposer d'indicateurs ou biomarqueurs de la perception des couleurs qui soient plus simples à obtenir et moins contraignants.

RESUME DE L'INVENTION

Les inventeurs ont eu l'idée de s'appuyer sur la réponse de la pupille à la variation de luminance. En effet, cette réponse (contraction ou dilatation de la pupille) étant plus lente que le nystagmus, les inventeurs ont pensé que des équipements et des traitements moins sophistiqués seraient alors suffisants.

Dans ce contexte, les inventeurs proposent tout d'abord un procédé de génération d'un indicateur ou biomarqueur de la perception des couleurs chez un sujet mammifère, comprenant les étapes suivantes :

soumettre le sujet mammifère à au moins un stimulus multicolore, typiquement bicolore dynamique comprenant l'affichage, sur un périphérique d'affichage (écran ou tout autre support visuel), d'un motif multicolore, typiquement bicolore dont au moins deux couleurs sont inversées périodiquement à une fréquence dite de marquage. Le motif multicolore affiche ainsi plusieurs couleurs (dont les deux qui s'inversent) à chaque instant donné,
contrôler une modification dans le temps d'au moins une des deux couleurs du motif multicolore lors de l'affichage du stimulus multicolore dynamique, pour faire varier la luminance affichée de cette couleur (généralement plusieurs fois). La modification de la couleur a pour vocation de faire varier dans le temps la luminance relative entre les deux couleurs (c'est-à-dire une différence de luminance réelle ou perçue),
acquérir, par un dispositif d'acquisition d'images, une réponse oscillatoire d'au moins une pupille du sujet mammifère, lors de l'affichage du stimulus multicolore dynamique, et
générer, à partir de la réponse acquise, un signal représentatif de la puissance de la réponse oscillatoire de la pupille en fonction de la modification dans le temps d'au moins une des deux couleurs (et plus généralement d'une différence de luminance entre les deux couleurs) lors de l'affichage du stimulus multicolore dynamique.

De façon classique, la réponse oscillatoire de la pupille consiste à mesurer l'évolution (constriction et dilatation) du diamètre de la pupille dans le temps. Avantageusement, les pupilles des deux yeux peuvent être analysées séparément ou en combinaison (par exemple au travers d'une moyenne).

Du fait de la réponse oscillatoire lente de la pupille, la fréquence de marquage utilisée est assez faible et, de plus, éloignée des autres fréquences physiologiques du sujet.

Par cet éloignement des fréquences physiologiques, le rapport signal/bruit de la réponse oscillatoire de la pupille se trouve être naturellement élevé. Il permet alors d'obtenir un signal représentatif de la puissance de la réponse oscillatoire qui représente fidèlement la réponse pupillaire, sans contamination. Ce signal représentatif de la réponse rétinienne et/ou perception (propre au sujet) relative des couleurs testées peut donc servir directement ou être lié directement à un indicateur ou biomarqueur fiable de la discrimination et/ou perception des couleurs.

Le signal généré peut alors être utilisé dans des applications médicales ou non-médicales, comme évoqué par la suite, et par exemple pour la détermination d'une iso-luminance chromatique entre deux couleurs testées.

En outre, par l'usage d'une fréquence de marquage faible, généralement de l'ordre de 0,1 Hz à 5 Hz, par exemple de 1,3 ou 1,4 Hz, des dispositifs d'acquisition vidéo conventionnels, typiquement des caméras 25 images par seconde (comme celles équipant les ordinateurs et téléphones mobiles), sont suffisants. Les traitements, notamment temps réel, des signaux acquis s'en trouvent alors substantiellement allégés.

Corrélativement, les inventeurs proposent un système de génération d'un indicateur ou biomarqueur de la perception des couleurs chez un sujet mammifère, comprenant :

un périphérique d'affichage,
un système informatique de stimulation du sujet mammifère par stimulus multicolore, typiquement bicolore dynamique, le système informatique commandant l'affichage, sur le périphérique d'affichage, d'un motif multicolore, typiquement bicolore dont au moins deux couleurs sont inversées périodiquement à une fréquence dite de marquage,
un contrôleur de couleurs configuré pour modifier dans le temps au moins une des deux couleurs du motif multicolore lors de l'affichage du stimulus multicolore dynamique, pour faire varier la luminance affichée de cette couleur,
un dispositif d'acquisition d'images pour acquérir une réponse oscillatoire d'au moins une pupille du sujet mammifère, lors de l'affichage du stimulus multicolore dynamique, et
un générateur d'indicateur ou biomarqueur configuré pour générer, à partir de la réponse acquise, un signal représentatif de la puissance de la réponse oscillatoire de la pupille en fonction de la modification dans le temps d'au moins une des deux couleurs lors de l'affichage du stimulus multicolore dynamique.

Ce système présente des avantages similaires à ceux du procédé décrit ci-dessus.

Des caractéristiques optionnelles du procédé sont par ailleurs définies par la suite. Le système peut également comprendre des moyens configurés pour mettre en oeuvre ces caractéristiques optionnelles.

Dans un mode de réalisation, le procédé comprend en outre une étape consistant à déterminer la configuration bicolore d'iso-luminance des deux couleurs (c'est-à-dire les valeurs de ces deux couleurs) du stimulus multicolore, typiquement bicolore dynamique correspondant à un minimum du signal représentatif de la puissance de la réponse oscillatoire de la pupille. Les inventeurs ont constaté qu'à raison de l'inversion périodique des deux couleurs dans le motif, la pupille oscille à la fréquence correspondante (de marquage) avec une réponse d'autant plus puissante que le différentiel perçu d'intensité des deux couleurs est important. Aussi, la détermination du minimum du signal représentatif de la puissance permet de déterminer l'iso-luminance chromatique perçue par le sujet pour les deux couleurs testées, c'est-à-dire lorsque la différence de luminance que l'on fait varier dans le temps entre les deux couleurs testées est minimale selon la perception du sujet. La correspondance d'iso-luminance chromatique peut être utilisée comme indicateur ou marqueur de la perception de la couleur chez un sujet. En effet, elle peut être comparée à celle obtenue pour d'autres sujets.

Les calculs mis en oeuvre pour la détermination de l'iso-luminance chromatique sont excessivement simplifiés par rapport aux techniques connues. En outre, la position du minimum dans le signal représentatif de la puissance est indépendante de l'unité utilisée pour mesurer la réponse oscillatoire de la pupille. Aussi, cette configuration permet de s'affranchir d'une calibration (ou étalonnage) du dispositif d'acquisition d'images.

Dans un mode de réalisation, l'autre couleur parmi les deux couleurs du motif multicolore est maintenue fixe pendant l'affichage du stimulus multicolore dynamique.

Dans un autre mode de réalisation, la fréquence de marquage est fonction du sujet. Notamment, le procédé peut comprendre en outre une étape préalable de détermination de la fréquence de marquage, comprenant la soumission du sujet à au moins un flash lumineux de calibration, la mesure d'un temps de réponse moyen de la pupille du sujet mammifère au flash lumineux de calibration, et la fixation de la fréquence de marquage en fonction du temps de réponse moyen mesuré.

Ces dispositions permettent notamment, à faible coût temporel, d'améliorer la fiabilité des mesures effectuées, par exemple la configuration d'iso-luminance chromatique pour les couleurs testées. En outre, elles permettent d'ajuster au mieux la durée du test (soumission au stimulus multicolore, typiquement bicolore dynamique), et notamment de la réduire.

En variante ou en combinaison, le motif multicolore utilisé est fonction du sujet, par exemple fonction de la réponse oscillatoire de la pupille aux flashs lumineux. Cela permet notamment de tenir compte d'une éventuelle pathologie du sujet qui altère la sensibilité spatiale des yeux du sujet. A titre d'exemple, le motif multicolore peut être positionné dans une zone préférentielle de l'écran compte tenu du sujet, par exemple le haut ou le bas de l'écran, ou encore un quart particulier de l'écran.

Dans un mode de réalisation, le contrôle de la modification dans le temps de la couleur comprend une modification graduelle par incréments (ou paliers). Cela permet d'acquérir des réponses oscillatoires stables, notamment sur une fenêtre glissante d'analyse. En particulier, une fréquence de modification de la couleur (par incrément) est inférieure à la fréquence de marquage. Préférentiellement, la fréquence de modification de la couleur est un sous-multiple de la fréquence de marquage. Cela permet d'avoir plusieurs alternances des mêmes deux couleurs dans le motif multicolore, typiquement bicolore affiché, et donc d'acquérir une réponse oscillatoire de meilleure qualité.

Dans un mode de réalisation, le sujet mammifère est soumis à deux stimuli multicolores, typiquement bicolores dynamiques successifs basés sur deux couples de couleurs différents. Cette disposition permet d'obtenir un indicateur relatif complet de la perception des couleurs chez le sujet. En effet, l'espace colorimétrique est généralement tri-dimensionnel (par exemple RGB, pour Red-Green-Blue, soit Rouge-Vert-Bleu) de telle sorte que la connaissance de la perception relative de deux couples de couleurs permet de déduire (par de simples calculs par exemple) la perception relative de l'ensemble des couples de couleurs. Cette perception relative est par exemple la correspondance d'iso-luminance chromatique.

Dans un mode de réalisation, l'affichage de couleurs sur le périphérique d'affichage est contrôlé à l'aide de triplets de valeurs Rouge-Vert-Bleu, et les deux couleurs du motif multicolore sont des couleurs pures pour lesquels deux des trois composantes Rouge, Vert, Bleu sont nulles. Ainsi, la modification dans le temps de la couleur se fait simplement par l'incrément progressif de la troisième composante non nulle.

Dans ce cas, les deux couples de couleurs indiqués ci-dessus sont choisis, dans l'espace RGB, parmi les couples RG, RB et GB, où R, G, B sont respectivement les couleurs rouge pure, verte pure et bleue pure.

Le signal représentatif de la puissance peut être formé de différentes manières selon le traitement appliqué au signal de réponse acquis.

Dans un premier mode de réalisation, le signal est un signal représentatif de la puissance d'oscillation de la pupille à la fréquence de marquage et/ou à une ou plusieurs de ses harmoniques, représentatif de l'évolution de la composante fréquentielle, à ladite fréquence de marquage et/ou à une ou plusieurs de ses harmoniques, de la réponse oscillatoire de la pupille. On entend, par « harmoniques », les multiples et/ou sous-multiples de la fréquence de marquage. Typiquement, on peut s'intéresser, outre la fréquence de marquage $F_{tag}$, à la demi-harmonique $F_{tag}/2$ et/ou à la double harmonique $2*F_{tag}$.

Le passage dans le domaine fréquentiel améliore la résistance de l'indicateur ou biomarqueur alors généré, au bruit. Par exemple, la génération du signal représentatif de la puissance d'oscillation de la pupille à la fréquence de marquage comporte l'application, à la réponse acquise, d'une transformée de Fourier rapide discrète sur une fenêtre temporelle glissante et la mémorisation, pour chaque fenêtre temporelle, de la valeur de la composante fréquentielle à la fréquence de marquage et/ou à une ou plusieurs de ses harmoniques du spectre fréquentiel obtenu. Ces traitements peuvent être avantageusement réalisés en temps réel, avec peu de ressources mémoires.

Notamment, la largeur de la fenêtre temporelle glissante est choisie au moins égale à la période associée à la fréquence de marquage, par exemple au moins le double de cette période.

Selon une autre caractéristique particulière, la fenêtre temporelle glissante est décalée d'un échantillon de la réponse acquise, à chaque nouvelle application de la transformée de Fourier rapide discrète. La précision temporelle des échantillons dépend généralement du dispositif d'acquisition d'images utilisé et de sa configuration. Avantageusement, la présente invention permet d'utiliser une fréquence d'échantillonnage de 25 Hz, correspondant à la définition d'une caméra classique (25 images par secondes). Ainsi, grâce à la disposition ci-dessus, le signal représentatif de la puissance d'oscillation généré a la meilleure définition possible compte tenu du dispositif d'acquisition utilisé.

Dans un mode de réalisation particulier, la génération du signal représentatif de la puissance d'oscillation de la pupille à la fréquence de marquage et/ou à une ou plusieurs de ses harmoniques comporte en outre l'approximation d'un signal formé des valeurs mémorisées de la composante fréquentielle à la fréquence de marquage et/ou à une ou plusieurs de ses harmoniques par au moins une fonction mathématique, par exemple une fonction par morceaux qui peut combiner une ou plusieurs sous-fonctions parmi une fonction affine, une fonction exponentielle. Des techniques classiques d'approximation et de sélection de chaque meilleure sous-fonction pour une partie du signal formé des valeurs mémorisées peuvent être utilisées. Le recours à une telle approximation permet d'obtenir un indicateur ou biomarqueur relativement simple, et donc de simplifier les traitements ultérieurs, par exemple l'usage de cet indicateur pour personnaliser des opérations informatiques ou pour évaluer l'évolution d'une pathologie ou l'efficacité d'un traitement contre une telle pathologie.

Dans un second mode de réalisation, la génération du signal représentatif de la puissance comporte la détermination d'une amplitude de variation du diamètre de la pupille en réponse à chaque inversion des couleurs du motif multicolore, typiquement bicolore, ledit signal représentatif de la puissance étant formé à partir des amplitudes ainsi déterminées. Ces amplitudes, c'est-à-dire les différences de diamètre de la pupille à chaque réponse pupillaire, sont en effet représentatives de la puissance de la réponse pupillaire, à un coefficient près (le temps de réponse de la pupille).

Toute autre méthode permettant d'obtenir un signal représentatif de la puissance de la réponse oscillaire (c'est-à-dire la réponse à chaque changement contrôlé dans l'affichage du motif multicolore) de la pupille peut être utilisée.

Pour l'ensemble de ces méthodes l'analyse peut être effectuée sans filtrage préalable particulier. Par ailleurs et avantageusement, la présente invention permet d'utiliser une fréquence d'échantillonnage de 25 Hz, correspondant à la définition d'une caméra classique (25 images par secondes). Ainsi, le signal représentatif de la puissance d'oscillation est généré avec la meilleure définition possible compte tenu du dispositif d'acquisition utilisé.

Dans un mode de réalisation, le procédé peut en outre comprendre la détermination du minimum du signal représentatif de la puissance ainsi généré, par exemple le minimum de la fonction par morceaux construite ci-dessus ou le minimum du signal des amplitudes d'oscillation de la pupille. Ce minimum permet alors d'identifier la configuration multicolore, typiquement bicolore d'iso-luminance perçue (ou les configurations multicolores d'iso-luminance si par exemple le motif inclut plusieurs couples de couleurs dont la luminance de l'une d'entre elles est modifiée dans le temps).

Dans un mode de réalisation, le procédé comprend en outre une étape de filtrage de la réponse oscillatoire acquise, l'étape de filtrage comprenant l'interpolation, préférentiellement linéaire, d'un signal de réponse oscillatoire lors d'un clignement d'œil. Cela permet de corriger des artéfacts ponctuels dans la courbe.

Les inventeurs ont constaté que la configuration d'iso-luminance obtenue par la méthode de marquage fréquentiel ci-dessus constitue une signature objective et robuste du sujet.

Or nombre de systèmes informatiques requièrent d'obtenir, de façon simple et rapide, des informations fiables et robustes d'un individu, et notamment de l'utilisateur, afin de piloter efficacement des processus informatiques. C'est le cas par exemple de processus d'identification ou authentification de l'utilisateur. Des techniques actuelles sont complexes et parfois contournables par le vol de l'information d'identification de l'utilisateur. C'est aussi le cas du paramétrage du système informatique qui doit être adapté au mieux à l'utilisateur, par exemple la calibration (étalonnage) d'écran.

Aussi, les inventeurs ont constaté que l'utilisation de cette configuration d'iso-luminance pour piloter de tels processus s'avère excessivement efficace. Ainsi, ce pilotage est rendu simple, rapide, robuste, et ne nécessitant pas de ressources complexes.

Dans ce contexte, les inventeurs proposent un procédé de contrôle personnalisé d'un processus dans un système informatique, comprenant les étapes suivantes :
déterminer au moins une information d'iso-luminance chromatique pour un individu comme ci-dessus. C'est-à-dire que la détermination d'une information d'iso-luminance comprend les étapes suivantes :

soumettre l'individu à au moins un stimulus multicolore, typiquement bicolore dynamique comprenant l'affichage, sur un périphérique d'affichage du système informatique, d'un motif multicolore, typiquement bicolore dont au moins deux couleurs sont inversées périodiquement à une fréquence dite de marquage. Le motif multicolore affiche ainsi plusieurs couleurs (dont les deux qui s'inversent) à chaque instant donné,

contrôler une modification dans le temps d'au moins une des deux couleurs du motif multicolore lors de l'affichage du stimulus multicolore dynamique, pour faire varier la luminance affichée de cette couleur. La modification de la couleur a pour vocation de faire varier dans le temps la luminance relative entre les deux couleurs (c'est-à-dire la différence de luminance perçue),

acquérir, par un dispositif d'acquisition d'images, une réponse oscillatoire d'au moins une pupille de l'individu, lors de l'affichage du stimulus multicolore dynamique,

générer, à partir de la réponse acquise, un signal représentatif de la puissance de la réponse oscillatoire de la pupille en fonction de la modification dans le temps d'au moins une des deux couleurs lors de l'affichage du stimulus multicolore dynamique, et

déterminer la configuration bicolore d'iso-luminance des deux couleurs dans le stimulus multicolore dynamique correspondant à un minimum du signal représentatif de la puissance. Il s'agit de la valeur desdites deux couleurs obtenues lorsque la différence de luminance que l'on fait varier dans le temps entre ces deux couleurs testées est minimale selon la perception du sujet, et

utiliser l'au moins une information d'iso-luminance chromatique ainsi déterminée comme donnée d'entrée du processus dans le système informatique.

Corrélativement, il est également proposé un système de contrôle personnalisé d'un processus dans un système informatique, comprenant :

un sous-système de détermination d'au moins une information d'iso-luminance chromatique pour un individu, et

un module de contrôle de processus configuré pour utiliser l'au moins une information d'iso-luminance chromatique ainsi déterminée comme donnée d'entrée du processus dans le système informatique.

Le sous-système de détermination d'une information d'iso-luminance comprend notamment :

un périphérique d'affichage,

un système informatique de stimulation de l'individu par stimulus multicolore, typiquement bicolore dynamique, le système informatique commandant l'affichage, sur le périphérique d'affichage, d'un motif multicolore, typiquement bicolore dont au moins deux couleurs sont inversées périodiquement à une fréquence dite de marquage,

un contrôleur de couleurs configuré pour modifier dans le temps au moins une des deux couleurs du motif multicolore lors de l'affichage du stimulus multicolore dynamique, pour faire varier la luminance affichée de cette couleur,

un dispositif d'acquisition d'images pour acquérir une réponse oscillatoire d'au moins une pupille de l'individu, lors de l'affichage du stimulus multicolore dynamique,

un générateur d'indicateur ou biomarqueur configuré pour générer, à partir de la réponse acquise, un signal représentatif de la puissance de la réponse oscillatoire de la pupille en fonction de la modification dans le temps d'au moins une des deux couleurs lors de l'affichage du stimulus multicolore dynamique, et

une unité de détermination d'iso-luminance configurée pour déterminer la configuration d'iso-luminance des deux couleurs dans le stimulus multicolore dynamique correspondant à un minimum du signal représentatif de la puissance.

Des caractéristiques optionnelles du procédé de contrôle personnalisé sont par ailleurs définies par la suite. Le système correspondant peut également comprendre des moyens configurés pour mettre en oeuvre ces caractéristiques optionnelles.

La calibration des écrans d'affichage pour obtenir une perception correcte des couleurs par l'individu (ici généralement l'utilisateur) est un sujet complexe, qui requiert généralement des équipements adaptés onéreux. Courant 2016, l'option « True Tone » a été mise en place sur des terminaux et tablettes mobiles Apple (nom commercial) ou équipés Android (nom commercial). Cette option permet l'adaptation dynamique de la balance des blancs, et ainsi de la luminance affichée, aux conditions environnementales (d'éclairage) de l'écran. La perception des couleurs par les utilisateurs est censée être améliorée, notamment pour que ces utilisateurs aient le même rendu affiché et donc la même perception d'une même image affichée lorsqu'ils utilisent différents écrans.

Cette adaptation dynamique n'est toutefois pas satisfaisante, car elle ne permet pas à différents utilisateurs d'avoir la même perception de la même image sur ces écrans. En effet, l'option « True Tone » n'est pas représentative de la perception de l'utilisateur car elle ne tient pas compte du capteur (œil) et des moyens de traitement (nerf optique, aires cérébrales visuelles) propres à l'utilisateur.

Dans un mode de réalisation, les inventeurs ont alors pensé appliquer ce procédé à la calibration d'un écran d'affichage du système informatique. Dans ce cas, l'affichage d'un pixel (plus généralement de tous les pixels) est corrigé en fonction de l'au moins une information d'iso-luminance chromatique déterminée. Il s'agit donc d'un procédé de calibration d'un écran d'affichage connecté à un système informatique, comprenant la détermination d'une information d'iso-luminance chromatique pour un utilisateur comme décrite ci-dessus, et la calibration des couleurs affichées sur l'écran en fonction de cette information d'iso-luminance chromatique déterminée.

Cette disposition permet ainsi d'ajuster l'affichage des pixels à chaque utilisateur de sorte que, quel que soit l'utilisateur, l'image affichée présente la même luminance perçue entre chaque couleur. Par exemple, une image parfaitement iso-luminante est perçue de la sorte par chaque utilisateur après calibration personnalisée. Aussi, plusieurs utilisateurs peuvent travailler sur une même image avec la même perception des couleurs alors qu'ils utilisent des écrans différents, à des emplacements parfois distants.

Dans un mode de réalisation, l'au moins une information d'iso-luminance chromatique comprend, pour au moins un couple de couleurs parmi les couples rouge-vert, rouge-bleu et vert-bleu de l'espace colorimétrique rouge-vert-bleu, une valeur déterminée pour une première couleur lorsque la deuxième couleur prend une valeur de référence. On peut en effet réaliser la calibration en considérant une couleur comme fixée à une référence (par exemple le rouge à (140,0,0)) et en utilisant le vert d'iso-luminance chromatique alors déterminée pour ajuster l'affichage de la composante verte (ou rouge) à l'écran par rapport au rouge (au vert).

De préférence, des couleurs pures (deux composantes nulles) sont utilisées.

Dans un mode de réalisation particulier, on obtient, pour une calibration initiale (par exemple par défaut) de l'écran d'affichage, une valeur par défaut d'iso-luminance chromatique de la première couleur lorsque la deuxième couleur prend la valeur de référence, et

la correction d'un pixel comprend l'ajustement (c'est-à-dire la modification de la valeur du pixel qui aurait dû être affichée sans calibration personnalisée selon l'invention) de la première ou deuxième ou des deux couleurs du pixel en fonction du différentiel de valeur entre la valeur par défaut d'iso-luminance chromatique et la valeur déterminée d'iso-luminance chromatique. Par exemple, si la calibration initiale définit une configuration d'iso-luminance associant la couleur rouge (140,0,0) à la couleur verte (0,140,0), la détermination d'une couleur verte d'iso-luminance (0,120,0) pour l'utilisateur (pour le couple rouge-vert) peut conduire à réduire la composante verte des pixels à afficher sur l'écran en fonction de l'écart de 20 unités ainsi déterminé ou d'augmenter la composante rouge d'autant.

On obtient ainsi une correction chromatique personnalisée d'un pixel (ou d'une image) simple à mettre en œuvre, pour converger vers une perception du pixel (ou de l'image) par cet utilisateur identique à la perception de la couleur par n'importe quel autre utilisateur bénéficiant également d'une correction chromatique personnalisée.

Selon une caractéristique optionnelle, la correction du pixel comprend la modification de la première ou deuxième couleur du pixel d'une valeur égale audit différentiel de valeur. Cette configuration est excessivement simple à mettre en œuvre.

Selon une variante, la correction du pixel comprend la modification de la première couleur du pixel d'une valeur qui est fonction à la fois du différentiel de valeur et de la distance de la deuxième couleur du pixel à la valeur de référence.

Notamment, cette fonction est préférentiellement linéaire par rapport à ladite distance entre la deuxième couleur du pixel et la valeur de référence. Cela permet d'ajuster efficacement la correction de sorte que les valeurs modifiées restent, sans traitement supplémentaire, dans l'espace colorimétrique utilisé.

Par exemple, la linéarité peut être déterminée par le fait que les deux extrémités de la plage de valeurs (correspondant par exemple aux couleurs (0,0,0) et (255,255,255)) sont des points invariants. Dans ce cas, la fonction est linéaire par morceaux de part et d'autre du point d'iso-luminance déterminé à la deuxième couleur de référence.

Dans un mode de réalisation, le procédé de contrôle personnalisé (ou plus précisément de calibration d'écran) comprend la détermination de deux informations d'iso-luminance chromatique pour deux couples différents de couleurs parmi les couples rouge-vert, rouge-bleu et vert-bleu de l'espace colorimétrique rouge-vert-bleu, chaque information chromatique d'iso-luminance comprenant une valeur déterminée pour une première couleur lorsque la deuxième couleur prend une valeur de référence. Cela permet de connaître les paramètres d'iso-luminance de l'utilisateur sur tout l'espace colorimétrique considéré, ici RGB. Aussi, par ces deux seules mesures, l'invention permet de calibrer entièrement l'écran d'affichage de sorte que la perception des couleurs de l'utilisateur soit la même que celle d'un autre utilisateur bénéficiant d'une calibration personnalisée selon les mêmes principes.

Selon une caractéristique particulière, la correction d'un pixel sur l'écran d'affichage comprend l'ajustement d'un triplet de couleurs (généralement RGB) définissant le pixel selon une calibration initiale de l'écran d'affichage, en fonction de deux différentiels de valeur obtenus pour les deux couples de couleurs, chaque différentiel de valeur représentant la différence entre une valeur par défaut d'iso-luminance chromatique pour la première couleur du couple de couleurs lorsque la deuxième couleur prend la valeur de référence et la valeur déterminée d'iso-luminance chromatique.

Reprenons l'exemple précédent où la mesure d'iso-luminance Rouge-Vert pour l'utilisateur a donné la configuration d'iso-luminance Rouge(140,0,0)-Vert(0,120,0) avec donc un décalage du vert de -20 unités. De même, une mesure d'iso-luminance est conduite pour l'utilisateur sur le couple Rouge-Bleu avec comme résultat la configuration d'iso-luminance Rouge(140,0,0)-Bleu(0,0,170), avec donc un décalage du bleu de +30 unités par rapport à la configuration d'iso-luminance par défaut Rouge(140,0,0)-Bleu(0,0,140). La correction d'affichage peut alors consister à réduire la composante verte de 20 unités (ou moins plus la composante rouge s'éloigne de 140) et à augmenter la composante bleu de 30 unités (ou moins plus la composante rouge s'éloigne de 140).

La perception identique des couleurs par différents utilisateurs est ainsi simple à obtenir.

Dans un mode de réalisation, l'ajustement du triplet de couleurs est effectué à valeur constante d'une de ses composantes couleurs. Cette configuration limite les calculs à opérer sur les composantes des pixels à afficher.

En variante, l'ajustement du triplet de couleurs est effectué à luminance du pixel constante. Cette configuration permet de conserver la luminance globale d'une image affichée après calibration personnalisée de l'écran selon les enseignements de l'invention. Généralement les trois composantes couleur sont ajustées en fonction des deux différentiels obtenus par les configurations d'iso-luminance.

Les systèmes d'identification ou authentification biométriques actuels ne sont pas suffisamment sûrs. Notamment, les informations biométriques demandées peuvent être reproduites ou ne requièrent pas que l'individu dont on veut vérifier l'identité par exemple soit vivant. Il est donc un besoin d'améliorer ces techniques d'identifications biométriques.

Aussi, les inventeurs ont constaté que l'application du procédé de contrôle personnalisé à une telle identification/authentification d'individu permet de surmonter des inconvénients des techniques connues. Dans ce procédé, le processus utilisant l'au moins une information d'iso-luminance chromatique est une procédure d'identification ou authentification de l'individu, par exemple auprès du système informatique. Il s'agit dans ce cas d'une procédé d'identification ou authentification d'un individu, comprenant la détermination d'au moins une information d'iso-luminance chromatique pour un individu comme décrite ci-dessus, et l'utilisation de cette information d'iso-luminance chromatique déterminée comme identifiant dans un processus d'identification ou d'authentification auprès du système informatique.

Comme l'information d'iso-luminance chromatique déterminée par l'invention requiert une réponse pupillaire oscillatoire de l'individu, ce dernier doit être vivant pour valider l'identification ou authentification. En outre, elle s'appuie sur des équipements peu complexes, comparativement aux capteurs d'empreintes digitales ou d'iris.

Dans un mode de réalisation, le procédé comprend en outre la comparaison de l'au moins une information d'iso-luminance chromatique déterminée avec au moins une information d'iso-luminance chromatique de référence correspondante, mémorisée en mémoire du système informatique en association avec un identifiant de l'individu, et l'identification ou authentification de l'individu n'est validée que si les informations d'iso-luminance correspondent.

Dans un mode de réalisation préféré, le procédé comprend la détermination de deux informations d'iso-luminance chromatique pour deux couples différents de couleurs parmi les couples rouge-vert, rouge-bleu et vert-bleu de l'espace colorimétrique rouge-vert-bleu, chaque information chromatique d'iso-luminance comprenant une valeur déterminée pour une première couleur lorsque la deuxième couleur prend une valeur de référence. Cela permet de connaître les paramètres d'iso-luminance de l'individu sur tout l'espace colorimétrique considéré, ici RGB, et ainsi d'en déduire, par calculs simples, n'importe quelle combinaison de couleurs d'iso-luminance nécessaire à la vérification d'identification/authentification.

En outre, cette vérification peut alors être effectuée sur plusieurs points de l'espace colorimétrique, améliorant la sécurisation d'accès au système informatique.

Dans un mode de réalisation, le procédé comprend en outre une étape préliminaire d'identification de l'individu pour récupérer l'au moins une information d'iso-luminance chromatique de référence associée à l'individu, avant ladite comparaison. Ainsi, la vérification selon l'invention à partir de la ou des configurations d'iso-luminance chromatique est complémentaire d'une identification plus classique. Cela permet notamment de limiter la précision nécessaire dans la détermination de cette ou ces configurations d'iso-luminance chromatique, et donc de réduire le temps nécessaire à l'identification/authentification.

Par exemple, l'étape préliminaire d'identification de l'individu comprend l'acquisition d'une information biométrique de l'individu, par exemple une empreinte digitale, une image de l'iris. En variante, un simple code/login peut être demandé.

Dans un mode de réalisation, le procédé comprend en outre la détermination, en fonction de l'identification de l'individu ainsi obtenue, d'au moins une plage restreinte (par rapport à une plage de valeurs possibles) de valeurs de modification pour modifier dans le temps la couleur à modifier du motif multicolore, typiquement bicolore lors de la détermination d'une information d'iso-luminance chromatique. Par exemple, l'identification de l'individu dont la valeur verte d'iso-luminance est fixée à 120 (pour un rouge à 140) peut permettre de réduire la plage de valeurs à tester à [80-160]. Cela réduit substantiellement la quantité de données à acquérir, de traitement à effectuer et donc le temps d'acquisition de la signature neurologique de l'individu.

Pour renforcer la sécurité du système, cette plage de valeurs réduite peut être générée aléatoirement à la volée, pour autant qu'elle inclue la valeur d'iso-luminance stockée en mémoire, plus une marge autour. En variante, elle peut être fixée en mémoire du système informatique, en association avec l'identifiant de l'individu.

## BREVE PRESENTATION DES FIGURES

D'autres particularités et avantages de l'invention apparaîtront encore dans la description ci-après, illustrée par les dessins ci-joints, dans lesquels :

- la **Figure 1** illustre un système de génération d'un indicateur ou biomarqueur de la perception des couleurs chez un sujet mammifère selon un mode de réalisation de l'invention ;
- la **Figure 2** illustre schématiquement un stimulus bicolore dynamique selon un mode de réalisation de l'invention ;
- la **Figure 3a** illustre un motif ou profil de modification d'une des couleurs du motif bicolore du stimulus de la **Figure 2** selon un mode de réalisation ;
- la **Figure 3b** représente un exemple d'une réponse oscillatoire d'une pupille en réponse à un stimulus bicolore dynamique du type celui de la **Figure 2** selon un mode de réalisation ;
- la **Figure 3c** illustre schématiquement un spectre fréquentiel issu de l'application d'une transformée de Fourier rapide discrète sur une fenêtre d'analyse du signal de la **Figure 3b** selon un mode de réalisation ;
- les **Figures 3d** et **3d'** illustrent des signaux représentatifs de la puissance d'oscillation de la pupille, et une fonction par morceaux les approximant, selon différents modes de réalisation ;
- la **Figure 3e** illustre la détermination d'une valeur d'iso-luminance chromatique sur le profil de modification de la **Figure 3a** selon un mode de réalisation ;
- la **Figure 4** illustre, par un organigramme, un procédé de génération d'un indicateur ou biomarqueur de la perception des couleurs chez un sujet mammifère selon des modes de réalisation de l'invention ; et
- la **Figure 5** illustre, par un organigramme, un procédé de calibration d'un écran d'affichage utilisant l'indicateur ou biomarqueur de type information d'iso-luminance déterminé par le procédé de la **Figure 4,** selon des modes de réalisation de l'invention ;
- la **Figure 6** illustre différents profils de correction des couleurs d'un pixel dans le procédé de la **Figure 5** ;
- la **Figure 7** illustre, par un organigramme, un procédé préalable de détermination et d'enregistrement d'informations d'identification/authentification d'un individu; et
- la **Figure 8** illustre, par un organigramme, un procédé d'identification ou authentification d'un individu à partir d'informations d'iso-luminance déterminées selon le procédé de la **Figure 4,** selon des modes de réalisation de l'invention.

## DESCRIPTION DETAILLEE DE MODES DE REALISATION DE L'INVENTION

La présente invention s'intéresse à la perception ou discrimination des couleurs chez les mammifères pour en faire un biomarqueur caractéristique de chaque sujet testé. Elle tire profit de la réponse oscillatoire pupillaire lente (entre 0,1 Hz à 5 Hz) d'un sujet mammifère à un stimulus lumineux oscillatoire pour générer une signature neurologique objective de la perception relative des couleurs par un sujet.

Une méthode par marquage fréquentiel de la pupille (« pupil frequency-tagging method » en langue anglo-saxonne) est utilisée à une fréquence de marquage $F_{tag}$ adaptée à la vitesse de réponse pupillaire, et qui avantageusement est éloignée des autres fréquences physiologiques associées aux mouvements de réponse de l'œil du sujet. A titre d'exemple $F_{tag}$ est de l'ordre de 1,3 à 1,4 Hz voire moins.

Cette méthode est robuste aux bruits résultant d'autres mouvements oculaires du sujet et ne nécessite pas de calibration de la part du sujet.

Cette méthode permet par exemple de déterminer l'iso-luminance chromatique perçue par un sujet à partir de la signature neurologique obtenue.

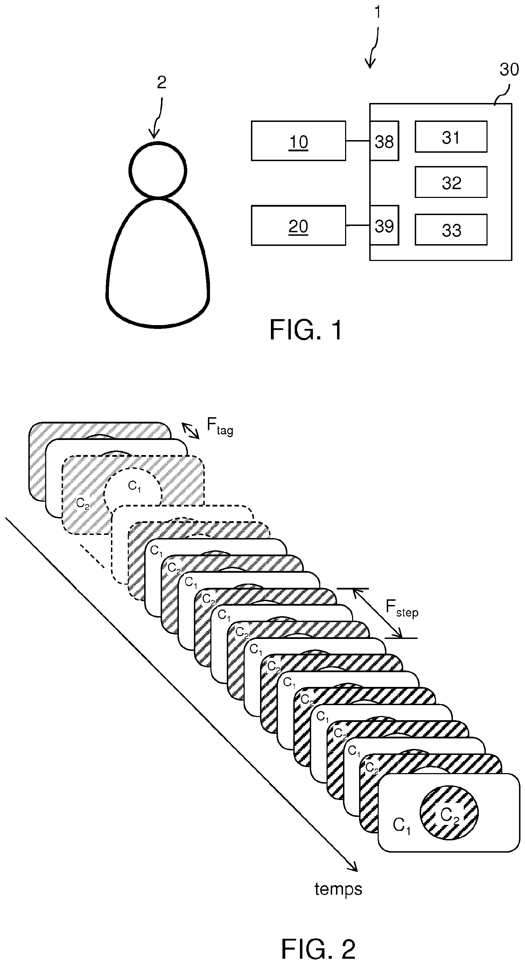

La **Figure 1** illustre un système 1 de génération d'un indicateur ou biomarqueur de la perception des couleurs chez un sujet mammifère 2.

Il comporte un écran d'affichage 10, un dispositif d'acquisition d'images 20 et un système informatique de commande et de traitement 30.

L'écran d'affichage 10 est une dalle de pixels, par exemple de résolution 1920x1080 pixels avec une fréquence d'affichage de par exemple 60 Hz, pilotées par une carte vidéo du système 30. Chaque pixel est formé de trois composantes couleurs sur 8 bits chacune (c'est-à-dire pouvant prendre des valeurs de 0 à 255) : Rouge, Vert, Bleu (RGB). Bien entendu d'autres définitions de pixels (nombre de bits par couleur, espace colorimétrique) peuvent être envisagées dans le cadre de la présente invention.

L'écran 10 peut être positionné centré face aux yeux du sujet, dans un plan perpendiculaire au regard, et à une distance fixe du sujet 2. Pour éviter tout mouvement du sujet 2, la tête de celui-ci peut être stabilisée en la faisant reposer par appui du menton et appui frontal sur des supports appropriés.

Le dispositif d'acquisition d'images 20 est typiquement une caméra sensible à l'infrarouge ou tout type de capteur permettant d'enregistrer un diamètre pupillaire, également disposée face au sujet afin de pouvoir acquérir des images d'une ou plusieurs pupilles du sujet 2.

Pour des raisons de simplification des explications, on se limitera par la suite à l'acquisition d'images d'une seule pupille du sujet 2. Bien entendu, des traitements similaires peuvent être réalisées sur l'acquisition d'images des deux pupilles, par exemple effectuer des moyennes ou corroborer les résultats obtenus pour une pupille par les résultats de l'autre pupille, ou enfin analyser l'altération d'une pupille relativement à l'autre.

Compte tenu de la fréquence de marquage $F_{tag}$ relativement faible, l'usage de caméra 20 sur étagère, du type 25 images/seconde, est possible. Notamment les caméras embarquées sur les dispositifs électroniques conventionnels (téléphones mobiles, ordinateurs, tablettes numériques, caméra portatives) peuvent être utilisées pour autant qu'elles soient sensibles aux infrarouges.

Bien entendu, des caméras disposant de fréquences d'acquisition supérieures à 25 im/s peuvent également être utilisées.

Lorsqu'une caméra opère sur un spectre fréquentiel plus large que celui de l'infrarouge, un filtrage (physique ou électronique) peut être prévu selon des techniques connues (et non décrites ici) afin d'obtenir en fin d'acquisition uniquement des images dans le spectre infrarouge ou à tout le moins permettre de déterminer le diamètre de la pupille observée.

Le système informatique de commande et de traitement 30 comporte un module informatique de stimulation du sujet mammifère par stimulus multicolore, typiquement bicolore dynamique 31, un contrôleur de couleurs 32 et un générateur d'indicateur ou biomarqueur 33. Ces différents éléments sont mis en œuvre par logiciel car il s'agit essentiellement de traitements visant à commander un affichage sur l'écran 10 ou à traiter des données acquises par le dispositif 20. A cette fin, le système 30 comporte également une carte vidéo classique 38 à laquelle est relié l'écran 10, et une carte d'acquisition vidéo classique 39 à laquelle est relié le dispositif d'acquisition 20.

Le système 30 peut en outre comprendre des moyens d'entrées/sorties (clavier, souris, carte réseau) pour permettre à un opérateur de paramétrer le système et de déclencher les processus et applications de l'invention.

Le module informatique de stimulation du sujet mammifère par stimulus multicolore, typiquement bicolore dynamique 31 commande l'affichage, sur l'écran 10, d'un motif multicolore, typiquement bicolore dont au moins deux couleurs sont inversées périodiquement à la fréquence de marquage $F_{tag}$.

Le motif multicolore en question affiche plusieurs couleurs à chaque instant donné. Au moins deux de ces couleurs sont alors inversées l'une l'autre périodiquement.

La **Figure 2** illustre schématiquement un exemple de motif bicolore formé des couleurs $C_1$ et $C_2$, un fond d'écran homogène d'une des couleurs (ici laissé en blanc pour une meilleure lisibilité) avec, en son centre, un cercle homogène de l'autre couleur (ici en hachuré pour une meilleure lisibilité). Le stimulus bicolore dynamique consiste à créer une alternance entre les deux couleurs $C_1$ et $C_2$ à la fréquence $F_{tag}$. Le motif bicolore peut être présenté sur un fond blanc par exemple.

Préférentiellement, les deux couleurs $C_1$ et $C_2$ du motif bicolore sont des couleurs pures, c'est-à-dire des couleurs pour lesquels deux des trois composantes Rouge, Vert, Bleu sont nulles. Dans l'espace colorimétrique RGB, $C_1$ et $C_2$ sont ainsi soit RG, soit RB, soit GB, où R, G, B sont respectivement les couleurs rouge pure $(r_i,0,0)$, verte pure $(0,g_i,0)$ et bleue pure $(0,0,b_i)$.

A noter que $C_1$ et $C_2$ peuvent être choisies comme étant la même couleur pure (RR, GG, BB), notamment pour des tests de contrôle. Bien entendu, d'autres choix de couples de couleurs $(C_1, C_2)$ n'impliquant pas nécessairement des couleurs pures peuvent être envisagés.

Le contrôleur de couleurs 32 est configuré pour modifier dans le temps au moins une des deux couleurs du motif multicolore, typiquement bicolore **(Figure 2)** lors de l'affichage du stimulus multicolore, typiquement bicolore dynamique sur l'écran 10, pour faire varier la luminance affichée de cette couleur (généralement plusieurs fois). Pour des couleurs pures, la variation de luminance est facile à obtenir, consistant simplement à modifier la seule composante ($r_i$ ou $g_i$ ou $b_i$) non nulle.

Cette modification d'une des deux couleurs a pour objectif de faire varier dans le temps la luminance relative entre les deux couleurs considérées, c'est-à-dire faire varier une différence de luminance entre ces deux couleurs, différence réelle ou perçue par le sujet.

Dans un mode de réalisation, l'autre couleur du motif, disons $C_i$, est maintenue fixe pendant l'affichage du stimulus bicolore dynamique. Cela signifie que pendant tout le test (l'ensemble des images affichées de la **Figure 2),** la couleur $C_1$ est affichée avec le même triplet RGB. Bien entendu, cette autre couleur peut également être modifiée dans le temps (par exemple en sens contraire de la première couleur). Ce qui importe ici est de déterminer le décalage entre les deux couleurs qui assure une iso-luminance perçue. En première approximation, ce décalage peut ensuite être appliqué à n'importe quelle valeur d'une des couleurs, pour obtenir la valeur de l'autre couleur en configuration d'iso-luminance.

La modification dans le temps de la couleur $C_2$ est préférentiellement réalisée par paliers croissants dans une plage de valeurs possibles (par exemple de 0 à 255 pour la seule composante modifiée) ou dans une plage de valeurs à tester, ou par paliers décroissants (par exemple de 255 à 0) pendant le test. Pour des couleurs non pures, la luminance résultante (différentes formules à partir des composantes RGB sont à la disposition de l'homme de l'art) sera choisie croissante ou décroissante pendant le test.

En variante, une modification de la couleur $C_2$ par dichotomie autour d'une première valeur d'iso-luminance déterminée lors d'un premier test (basé selon les techniques de la présente invention ou selon d'autres méthodes) peut être envisagée.

D'autres exemples de motifs multicolores peuvent être utilisés, par exemple incluant un plus grand nombre de couleurs ou un motif multicolore affichant deux ou plus couples différents de couleurs qui sont inversées deux à deux comme expliqué par exemple ci-dessus. Egalement, des motifs spatialement différents peuvent être envisagés dans la zone d'affichage sur l'écran 10, par exemple un affichage sur l'un ou l'autre des quarts d'écran ou bien encore un affichage sur la partie haute (ou droite) ou basse (ou gauche) de l'écran.

Pour simplifier les explications qui suivent, il est fait référence principalement à un motif bicolore dont les deux couleurs affichées simultanément sont inversées (lequel peut par exemple être affiché sur un fond uniforme, résultant en définitive en un affichage multicolore).

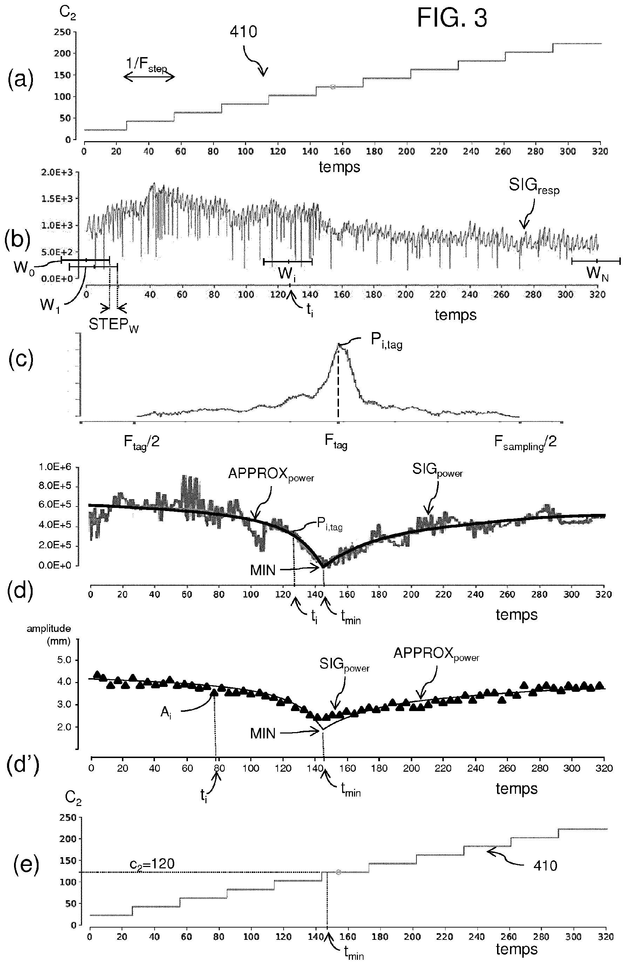

La **Figure 3a** illustre un exemple de contrôle de la modification dans le temps de la couleur $C_2$ consistant en une modification graduelle par incréments (ou escaliers). La différence de luminance entre les deux couleurs varie ainsi dans le temps. Dans cet exemple, on fait varier la composante non nulle de la couleur $C_2$ entre 20 et 220 (les extrêmes ne sont pas significatifs), par paliers de 20 unités. Bien entendu, d'autres plages testées, avec d'autres paliers peuvent être utilisés pour réduire ou allonger le temps de test, mais également réduire ou améliorer la précision de la détermination par exemple de l'iso-luminance chromatique.

Notamment, une précision plus importante (plage plus étroite et paliers plus petits) peut être utilisée, dans un second test, autour d'une valeur approximative d'iso-luminance (c'est-à-dire la valeur de $C_2$ ayant la même luminance perçue par le sujet 2 que la valeur fixe de $C_1$) déterminée au préalable lors d'un premier test (basé selon les techniques de la présente invention ou selon d'autres méthodes).

Comme illustré sur la **Figure 2,** la fréquence $F_{step}$ d'incrément de la valeur de $C_2$ est choisie inférieure à la fréquence de marquage $F_{tag}$, par exemple 3 fois inférieure **(Figure 2** - signifiant que trois images affichées consécutives ont la même valeur de couleur $C_2$), 10 fois inférieure environ **(Figure 3a)** voire plus, ou éventuellement un sous-multiple de $F_{tag}$.

De retour à la **Figure 1,** le générateur d'indicateur ou biomarqueur 33 est configuré pour générer, à partir d'une réponse oscillatoire $SIG_{resp}$ d'au moins une pupille du sujet 2 acquise par le dispositif d'acquisition 20, un signal de puissance d'oscillation $SIG_{power}$ de la pupille. Un exemple de réponse oscillatoire est illustré en **Figure 3b,** alors que des exemples de signaux de puissance d'oscillation de la pupille sont illustrés en **Figures 3d** et **3d'.**

Ce signal $SIG_{power}$ est représentatif de la puissance de la réponse oscillatoire de la pupille en fonction de la modification dans le temps **(Figure 3a)** d'au moins une des couleurs lors de l'affichage du stimulus bicolore dynamique.

Différents modes de réalisation peuvent être envisagés pour obtenir ce signal $SIG_{power}$.

Une première méthode rapide consiste à déterminer une amplitude $A_i$ de variation du diamètre de la pupille en réponse à chaque inversion (à $t_i$) des couleurs du motif bicolore. Chaque amplitude $A_i$ quantifie la modification du diamètre de la pupille lors de la réponse pupillaire. Une telle amplitude est représentative de la puissance de la réponse pupillaire, à un coefficient près (le temps de réponse de la pupille). Le signal $SIG_{power}$ est alors formé des amplitudes ainsi déterminées (voir **Figure 3d'** dans laquelle les triangles représentent schématiquement chaque amplitude $A_i(t_i)$ déterminée).

Une deuxième méthode plus résistante au bruit consiste à travailler dans le domaine fréquentiel de la réponse oscillatoire $SIG_{resp}$. Dans ce cas, le signal $SIG_{power}$ est préférentiellement un signal représentatif de la puissance d'oscillation de la pupille à la fréquence de marquage, représentatif de l'évolution de la composante fréquentielle $P_{i,tag}$, à ladite fréquence de marquage $F_{tag}$, de la réponse oscillatoire $SIG_{resp}$ de la pupille en fonction de la modification dans le temps **(Figure 3a)** de la couleur $C_2$ lors de l'affichage du stimulus bicolore dynamique **(Figure 2).** Le signal $SIG_{power}$ est alors formé des composantes fréquentielles $P_{i,tag}$ ainsi déterminées (voir **Figure 3d).**

Il est à noter que la détermination de ces composantes fréquentielles ne requiert pas nécessairement l'acquisition des réponses oscillatoires complètes à chaque inversion des couleurs. En effet, des techniques connues permettent d'obtenir ces composants fréquentielles à partir d'un cycle partiel de la réponse oscillatoire de la pupille. Dans un mode de réalisation préféré, on s'appuie sur des réponses oscillatoires complètes.

En variante ou en combinaison avec la première harmonique $F_{tag}$, il est possible de s'intéresser aux harmoniques de la fréquence de marquage $F_{tag}$, notamment les sous-harmoniques telles que la demi-harmonique $F_{tag}/2$ et/ou les harmoniques multiples telles que la double ou deuxième harmonique $2*F_{tag}$. Dans ce cas, chaque harmonique est traitée séparément comme décrit par la suite, leurs résultats pouvant être combinés (au travers d'une moyenne ou pour ajuster le résultat de la première harmonique, etc.) pour obtenir notamment une unique configuration bicolore d'iso-luminance.

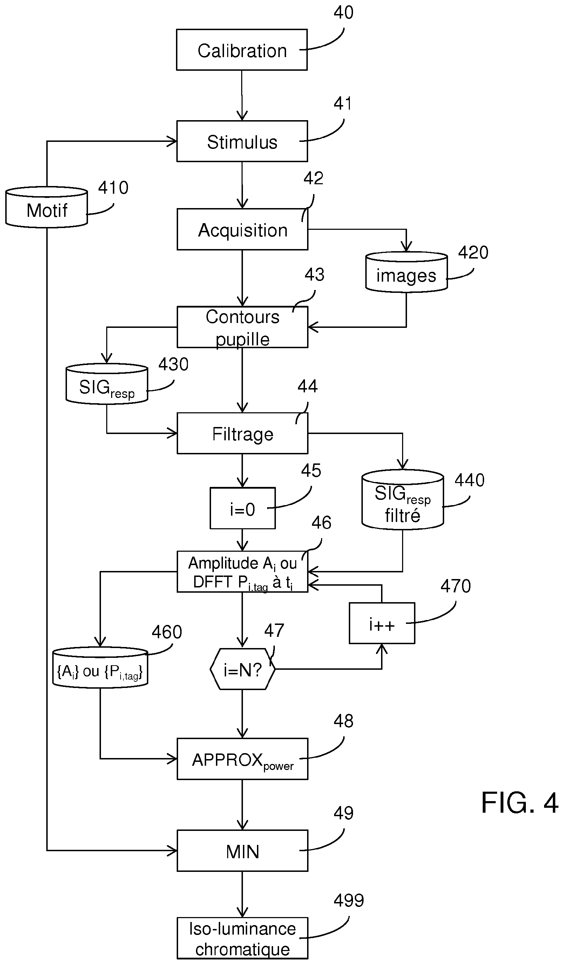

Ainsi, à partir du signal $SIG_{power}$ de n'importe quelle harmonique traitée, le générateur d'indicateur ou biomarqueur 33 peut notamment déterminer l'iso-luminance de $C_2$ par rapport à $C_1$ (fixe). Comme expliqué par la suite en référence à la **Figure 4** en lien avec les **Figures 3d** et **3e,** cette iso-luminance chromatique correspond à la configuration bicolore d'iso-luminance du stimulus bicolore dynamique correspondant à un minimum du signal $SIG_{power}$. En effet, l'amplitude de la réponse pupillaire et donc sa puissance d'oscillation suivent de près les variations de luminance du stimulus affiché. Elles sont donc minimales lorsque les variations de luminance sont minimales, c'est-à-dire lors de l'iso-luminance chromatique perçue par le sujet du motif bicolore affiché.

En référence maintenant à la **Figure 4,** on décrit un exemple de procédé de génération d'un indicateur ou biomarqueur de la perception des couleurs chez un sujet mammifère selon les enseignements de l'invention.

Au démarrage du système 1, une étape optionnelle 40 permet de calibrer le système 1 au sujet 2 testé. En particulier cette étape a pour but de déterminer la fréquence $F_{tag}$ en fonction du sujet 2 et/ou le motif à utiliser. En effet, le temps de réponse pupillaire varie, parfois fortement, d'un sujet à l'autre. Aussi, pour réduire la durée du test ou garantir des mesures pertinentes, il est préférable que $F_{tag}$ soit aussi grande que possible tout en évitant qu'elle ne dépasse la vitesse de réponse pupillaire du sujet.

Par ailleurs, un motif différencié spatialement par rapport au point de fixation du sujet peut être envisagé afin d'isoler des atteintes affectant spécifiquement certaines zones de la rétine du sujet. Par exemple, une différence de perception de couleur haut-bas peut exister pour des patients atteints d'Alzheimer, alors que des zones particulières sont difficilement perçues par des patients atteints localement de DMLA (ces zones correspondants aux parties dégénérées dans la rétine compte tenu de la pathologie).

L'étape 40 de calibration consiste de façon générale à enregistrer la réponse pupillaire du sujet (généralement en constriction), pour en déduire un temps de réponse pupillaire et ainsi une fréquence $F_{tag}$ à utiliser (par exemple à l'aide d'une table de correspondance), et/ou une zone de meilleure sensibilité du sujet définissant une zone préférentielle d'affichage du motif bicolore.

A titre d'exemple, le sujet 2 peut être soumis à au moins un flash lumineux de calibration commandé par le système 30 et affiché sur l'écran 10 (par exemple un écran blanc soudain à partir d'un écran sombre ou une zone blanche soudaine dans l'écran sombre, l'emplacement de cette zone blanche pouvant varier lors du test de calibration).

Une acquisition, par le dispositif 20, des images de la ou des réponses pupillaires du sujet 2 (au flash ou aux flashes multiples) permet d'obtenir un signal représentatif de la variation (constriction) de la pupille (notamment de son diamètre), à partir duquel le temps de réponse (éventuellement moyen sur plusieurs réponses) de la pupille peut être mesuré par des techniques classiques (par exemple le temps mis, à compter du flash, pour atteindre 90% de la constriction en réponse). Classiquement, le temps de réponse pupillaire d'un individu est de l'ordre de 0,5 secondes à 2 secondes.

A partir de ce temps de réponse mesuré ou calculé, l'étape 40 fixe la fréquence $F_{tag}$. Ce peut être fait à l'aide d'une table de correspondances (qui associe, à des plages de temps de réponse, des valeurs de $F_{tag}$ respectives) afin d'avoir un nombre limité de valeurs de fréquences de marquage. En variance, la période associée à la fréquence de marquage $F_{tag}$ peut être mise au double du temps de réponse ou à un autre multiple du temps de réponse mesuré.

Par défaut, une valeur $F_{tag}$ d'environ 1,3 à 1,4 Hz peut être utilisée.

Egalement à partir de réponses pupillaires à l'affichage d'un flash en plusieurs zones de l'écran, alors que le sujet conserve un point de fixation, le dispositif détermine une zone de meilleure sensibilité du sujet. Cette zone peut alors être utilisée pour l'affichage du motif bicolore sur une partie seulement de l'écran. Aussi, des motifs spatialement différenciés sont finalement affichés en fonction des sujets concernés.

A l'étape 41, le sujet 2 est soumis à au moins un stimulus multicolore, typiquement bicolore dynamique.

Bien que la description qui suit s'intéresse à la soumission du sujet à un seul stimulus bicolore, il peut être prévu de le soumettre successivement à deux stimuli bicolores dynamiques successifs basés par exemple sur deux couples de couleurs différents, afin notamment d'obtenir une perception relative des couleurs du sujet sur l'ensemble de l'espace colorimétrique tridimensionnel RGB. A titre d'exemple, un test déterminant l'iso-luminance chromatique du couple RG pour le sujet peut être combiné à un deuxième test ultérieur déterminant l'iso-luminance chromatique du couple GB. La perception relative entre les couleurs Rouge et Bleu peut alors se déduire directement, de sorte que la perception relative de toutes les couleurs dans l'espace colorimétrique est connue pour le sujet.

Egalement, bien que le stimulus utilisé ici soit bicolore au sens où seulement deux couleurs sont interverties, l'une d'entre elles au moins variant dans le temps, il peut être prévu que le stimulus multicolore utilisé comprennent plusieurs couples différents de couleurs interverties à la fréquence $F_{tag}$, affichés simultanément sur le même écran.

Lors de l'étape 41, le module informatique de stimulation du sujet mammifère par stimulus bicolore dynamique 31 commande l'affichage du motif bicolore éventuellement dans une zone préférentielle d'affichage et l'inversion périodique des deux couleurs $C_1$ et $C_2$ à la fréquence $F_{tag}$ **(Figure 2).** Dans le même temps, la luminance de la couleur $C_2$ est modifiée dans le temps selon un profil de modification 410, sous le contrôle du contrôleur de couleurs 32. Le profil de modification peut être incrémental comme montré sur la **Figure 3a** pour la composante non nulle de $C_2$.

A l'étape 42, le dispositif d'acquisition d'images 20 filme et acquiert des images, dans le spectre infrarouge, de la pupille surveillée du sujet 2. Ces images sont transmises au système 30 et stockées en mémoire 420. L'acquisition est réalisée à une fréquence d'échantillonnage $F_{sampling}$ de 25 à 1000 images par seconde, selon les capacités du dispositif 20.

A l'étape 43, les images acquises sont traitées à l'aide d'un algorithme de détection de contours de la pupille et de mesure du diamètre de la pupille. Ces mesures permettent de dresser un signal de réponse oscillatoire $SIG_{resp}$ de la pupille lors de l'affichage du stimulus bicolore dynamique, représentant l'évolution du diamètre de la pupille dans le temps.

La **Figure 3b** illustre un exemple de réponse oscillatoire $SIG_{resp}$ de la pupille à un stimulus bicolore dynamique Rouge-Vert, dont la couleur rouge pure est fixée à (140,0,0) et la couleur verte pure suit la courbe de la **Figure 3a** ($F_{step}$=0,0345 soit des paliers d'environ 29 secondes). La fréquence $F_{tag}$ est fixée à 0,345 Hz.

La courbe $SIG_{resp}$ ainsi obtenue est stockée en mémoire 430 du système 30.

A noter que les traitements de détection de contours et de génération de $SIG_{resp}$ peuvent être embarqués dans le dispositif d'acquisition 20 afin de réduire le volume de données à transmettre au système informatique 30.

La réponse oscillatoire $SIG_{resp}$ peut être exploitée telle quelle aux étapes 45 à 49 décrites ci-dessous. En effet, l'utilisation de la fréquence $F_{tag}$, éloignée des fréquences de bruits physiologiques, garantit une bonne robustesse de cette réponse.

Il peut cependant être prévu, de façon optionnelle, un filtrage de cette réponse aux fins notamment de supprimer des artéfacts dans le signal.

Dans ce cas, le procédé se poursuit à l'étape optionnelle 44 de filtrage de la réponse oscillatoire $SIG_{resp}$ acquise. Ce filtrage a notamment pour objectif de supprimer du bruit dans le signal, par exemple le bruit résultant d'un clignement de l'œil où aucune pupille n'a pu être détectée par l'algorithme de détection des contours).

Dans un mode de réalisation, l'étape 44 consiste à interpoler, préférentiellement de façon linéaire, le signal de réponse oscillatoire $SIG_{resp}$ lors d'un ou plusieurs clignements d'œil, c'est-à-dire lorsque l'algorithme de détection de contours n'a pas pu détecter de pupille dans les images. Des techniques classiques d'interpolation peuvent être utilisées.

A titre d'exemple, un filtrage basé sur la méthode Savitzky-Golay peut être mis en œuvre.

Le signal ainsi filtré est conservé en mémoire 440 du système 30.

C'est à partir de ce signal $SIG_{resp}$ filtré ou non que le signal $SIG_{power}$ est généré.

Dans le premier mode de réalisation évoqué ci-dessus, le générateur 33 peut déterminer les instants $t_i$ d'inversion des couleurs dans le stimulus bicolore affiché, puis déterminer dans chaque réponse oscillatoire correspondante, l'amplitude $A_i(t_i)$ de la variation du diamètre pupillaire du sujet 2.

Il peut s'agit simplement de déterminer la différence entre la valeur du diamètre au moment de l'inversion de couleur et la valeur extrémale opposée (maximale ou minimale) du diamètre dans une fenêtre temporelle correspondant sensiblement au temps de réponse du sujet.

Bien entendu, d'autres méthodes d'évaluation de l'amplitude peuvent être envisagées, comme par exemple utiliser un pourcentage de la valeur extrémale du diamètre ou la valeur du diamètre à 90% (ou moins) de la réponse.

L'ensemble des amplitudes calculées, associées aux instants respectifs d'inversion des couleurs, forment le signal $SIG_{power}$ **(Figure 3d')** utilisé aux étapes 48 et 49 ci-dessous.

Dans le deuxième mode de réalisation basé sur le domaine fréquentiel, le signal $SIG_{resp}$ obtenu (éventuellement filtré) est transformé dans le domaine spectral pour analyser l'évolution de la composante fréquentielle $F_{tag}$ et/ou de ses harmoniques. Pour ce faire, on applique au signal $SIG_{resp}$ une transformée de Fourier rapide discrète (DFFT) sur une fenêtre temporelle glissante $W_i$. On obtient ainsi un spectre fréquentiel correspondant à la portion de signal analysée par la fenêtre. Puis, on mémorise, pour chaque fenêtre temporelle d'analyse $W_i$, la valeur $P_{i,tag}$ de la composante fréquentielle à la fréquence $F_{tag}$ (et/ou d'une ou plusieurs harmoniques).

La suite de la description s'intéresse à la composante fréquentielle $F_{tag}$. Une approche similaire peut être utilisée pour traiter n'importe quelle harmonique d'intérêt.

La **Figure 3b** illustre les fenêtres temporelles d'analyse $W_i$ utilisées depuis la première fenêtre $W_0$ jusqu'à la dernière fenêtre $W_N$. Pour assurer la présence de la composante fréquentielle à $F_{tag}$ dans le spectre, la largeur de la fenêtre temporelle glissante $W_i$ est au moins égale à la période $T_{tag}$ associée à la fréquence de marquage $F_{tag}$. Dans l'exemple de la figure, on choisit par exemple une largeur égale à $2*T_{Tag}$ (ou supérieure). Ainsi, dès la fenêtre $W_0$, centrée sur $t_0$, une composante $P_{0,tag}$ non nulle peut être obtenue.

Chaque fenêtre d'analyse $W_i$ est centrée sur (donc associée à) l'instant $t_i$. Préférentiellement, on associe une fenêtre $W_i$ à chaque instant d'échantillonnage, afin d'obtenir la résolution maximale du signal représentatif de la puissance oscillatoire formé des valeurs $\{P_{i,tag}\}$. Dans ce cas, la fenêtre temporelle glissante W est décalée d'un échantillon de la réponse $SIG_{resp}$ acquise, à chaque nouvelle application de la transformée de Fourier rapide. Bien entendu, le pas $STEP_W$ de décalage de la fenêtre d'analyse W peut être supérieur à une seule période d'échantillonnage ($T_{sampling}$=1/$F_{sampling}$) utilisée dans cet exemple. Un pas $STEP_W$ fixé à plusieurs périodes d'échantillonnage permet de réduire les calculs et la taille mémoire requise.

Pour obtenir un signal représentatif de la puissance d'oscillation $SIG_{power}$ de la pupille à la fréquence $F_{tag}$ représentatif de l'évolution de la composante fréquentielle $F_{tag}$ de la réponse oscillatoire $SIG_{resp}$ de la pupille, le procédé peut tout d'abord comprendre l'initialisation d'une variable 'i' à zéro (pour traiter chaque échantillon) à l'étape 45.

Puis, la DFFT est appliquée à la portion du signal $SIG_{resp}$ définie par la fenêtre temporelle courante $W_i$ centrée sur l'échantillon 'i' à traiter (c'est-à-dire $t_i$). La **Figure 3c** illustre un exemple schématique de spectre fréquentiel obtenu.

On notera l'absence de composantes fréquentielles en deçà de la fréquence correspondant à la largeur de la fenêtre $W_i$ et au-delà de la demi-fréquence d'échantillonnage (critère de Nyquist). Des composantes d'harmoniques de $F_{tag}$ peuvent donc être présentes.

Ce graphique montre que la composante fréquentielle $P_{i,tag}$ à $F_{tag}$ présente un excellent rapport signal/bruit, du fait que $F_{tag}$ est éloignée des fréquences physiologiques du sujet 2 éventuellement parasitaires. La valeur $P_{i,tag}$ est obtenue et mémorisée en mémoire 460. Il s'agit de l'étape 46.

Il est entendu que la DFFT, puisqu'elle opère sur des valeurs discrètes (échantillons), ne produit pas nécessairement de composante fréquentielle exactement à la fréquence $F_{tag}$. Aussi, la valeur $P_{i,tag}$ peut être celle de la composante fréquentielle obtenue la plus proche de $F_{tag}$, ou une approximation (linéaire par exemple) entre les deux (ou plus) composantes fréquentielles entourant $F_{tag}$, ou encore une moyenne de deux ou plus composantes fréquentielles entourant $F_{tag}$.

A noter que l'utilisation d'une fréquence d'échantillonnage $F_{sampling}$ et/ou d'une taille de fenêtre W aussi grandes que possible accroît le nombre d'échantillons fréquentiels dans le spectre, notamment autour de $F_{tag}$.

Après l'étape 46, on détermine si toutes les DFFT ont été réalisées (test 47 vérifiant que i=N, nombre d'échantillons à traiter sur la période de test), sinon on incrémente la variable 'i' (étape 470) pour réaliser la DFFT du signal sur la fenêtre $W_i$ suivante.

Lorsque la DFFT a été appliquée à l'aide de toutes les fenêtres (c'est-à-dire sur tous les morceaux de la réponse $SIG_{resp}$), la mémoire 460 stocke l'ensemble des valeurs $P_{i,tag}$ qui forment ensemble le signal $SIG_{power}$ de la pupille à la fréquence $F_{tag}$ (voir **Figure 3d).**

Le signal $SIG_{power}$ généré selon l'une quelconque des méthodes constitue déjà un indicateur ou biomarqueur de la perception des couleurs chez le sujet 2. Il s'agit en effet d'une signature neurologique robuste du sujet 2. Comme on le verra par la suite, l'analyse de son évolution dans le temps peut permettre de détecter une pathologie ou une aggravation de la pathologie. C'est le cas par exemple de la sclérose en plaques.

Des étapes optionnelles 48 et 49 permettent de raffiner cet indicateur ou biomarqueur. La forme générale du signal $SIG_{power}$ montre une zone centrale plus basse que les zones latérales.

A l'étape optionnelle 48, un algorithme d'approximation du signal $SIG_{power}$ est mis en place afin notamment de faire correspondre ce signal à une fonction mathématique. Notamment, on cherche à faire correspondre le signal à une fonction par morceaux, où chaque morceau peut correspondre à une fonction affine, une fonction exponentielle ou toute autre fonction classique.

Dans l'exemple de la **Figure 3d** on utilise une fonction exponentielle par morceaux (deux morceaux) $APPROX_{power}$ afin de correspondre à la partie globalement décroissante du signal (à gauche) et à la partie globalement croissante du signal (à droite). Des techniques classiques d'approximation par mise en correspondance peuvent être utilisées, sans avoir à les détailler ici.

Le signal $APPROX_{power}$ ainsi obtenu peut également être utilisé comme indicateur ou biomarqueur de la perception des couleurs chez le sujet 2.

L'étape 49 consiste alors à déterminer la valeur $c_2$ de la couleur $C_2$ qui minimise le signal $SIG_{power}$ ou son approximation $APPROX_{power}$. Cela passe par la détermination du minimum MIN du signal $SIG_{power}$ ou $APPROX_{power}$, selon des techniques classiques et la détermination de la valeur de $C_2$ à l'instant $t_{min}$ correspondant à ce minimum de signal, à l'aide du profil de modification 410 **(Figure 3a** reprise en **Figure 3e).**

Dans l'exemple présent, la valeur $c_2(t_{min})=120$ est obtenue pour $C_2$. En d'autres termes, le sujet 2 perçoit une iso-luminance chromatique relative entre les couleurs pures rouge et verte pour les valeurs $C_1=(140,0,0)$ et $C_2=(0,120,0)$ pour l'exemple montré.

La configuration d'iso-luminance 499 ainsi obtenue peut servir d'indicateur ou biomarqueur de la perception des couleurs chez le sujet 2.

Cette configuration peut être combinée avec une ou plusieurs autres configurations correspondantes d'iso-luminance déterminées à partir d'une ou plusieurs harmoniques de $F_{tag}$.

Il est à noter que dans le cas où le stimulus multicolore dynamique utilisé comporte plusieurs couples différents de couleurs que l'on inverse à la fréquence $F_{tag}$, le minimum MIN du signal $SIG_{power}$ ou $APPROX_{power}$ correspond à une configuration d'iso-luminance globale des différents couples de couleurs, sans que cette configuration ne corresponde spécifiquement à une configuration d'iso-luminance de chaque couple de couleurs pris isolément.

Dans un mode de réalisation, la configuration d'iso-luminance obtenue peut être confirmée en reproduisant le test avec un profil de modification 410 inverse (décroissant par paliers de 220 à 20 par exemple). Notamment, une moyenne des deux valeurs $c_2$ obtenues lors de ces deux tests peut être calculée pour représenter la configuration moyenne d'iso-luminance du sujet 2 pour les couleurs $C_1$ et $C_2$.

La méthode ci-dessus a été testée sur 4 sujets humains pendant 14 sessions de 45 minutes et 7 sujets mammifères non-humains pendant une session de 20 minutes. La fréquence de marquage retenue a été 0,345 Hz, adaptée à l'ensemble des sujets étudiés. Le motif bicolore utilisé a été celui de la **Figure 2,** avec une couleur fixe et l'autre variant par escalier (comme illustré schématiquement sur la **Figure 3).** L'écran plat 10, de définition 1920x1080 pixels, 8 bits par couleur, 60 Hz a été fixé à une distance de 67 cm des sujets.

Les inventeurs ont bien constaté, pour l'ensemble des sujets, une réduction maximale de la puissance des oscillations pupillaires induites par les variations de luminance du stimulus à la configuration d'iso-luminance du motif bicolore testé.

Les inventeurs ont constaté que ce procédé d'obtention d'une configuration d'iso-luminance, signature personnelle d'un utilisateur, présente des avantages indéniables pour le pilotage ou contrôle personnalisé de processus informatique.

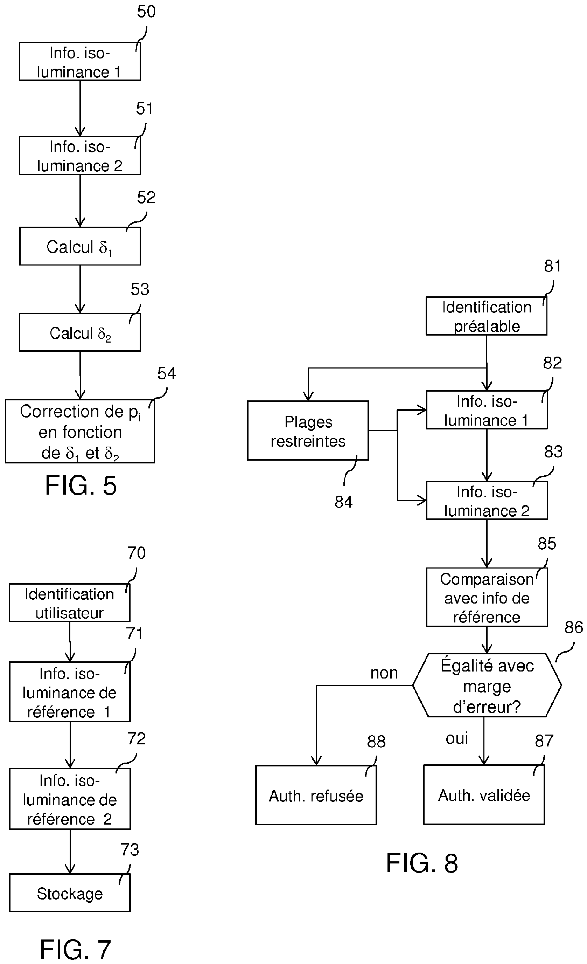

Les **Figures 5** à **8** illustrent plusieurs modes de réalisation d'un tel contrôle personnalisé, et par voie de conséquence plusieurs applications de la configuration d'iso-luminance dans des domaines non nécessairement médicaux.

D'une façon générale, le contrôle personnalisé d'un processus dans un système informatique, selon l'invention, comprend les étapes suivantes :

déterminer au moins une information d'iso-luminance chromatique pour un utilisateur du système informatique en utilisant les enseignements décrits ci-dessus, par exemple le procédé de la **Figure 4.** Notamment, une configuration d'iso-luminance du stimulus multicolore, typiquement bicolore dynamique est déterminée correspondant à un minimum du signal notamment $SIG_{power}$ ; et

utiliser l'au moins une information d'iso-luminance chromatique ainsi déterminée comme donnée d'entrée du processus dans le système informatique.

La **Figure 5** illustre l'application de la configuration d'iso-luminance à la calibration d'un écran d'affichage du système informatique. Il peut par exemple s'agir de calibrer l'écran 10 **(Figure 1)** en fonction de l'utilisateur 2, pour lui permettre d'afficher une image et d'en percevoir les couleurs de la même façon qu'un autre utilisateur le ferait sur la même image avec un autre écran calibré selon les mêmes techniques.

Un avantage de cette méthode est qu'elle peut être mise en œuvre sur des systèmes numériques grands publics dotés de caméra moyenne résolution : écran d'ordinateurs, ordinateurs portables, tablettes numériques, smartphones, etc., pour calibrer leurs écrans. Elle ne requiert en effet pas de composants complexes.

Le procédé illustré comprend, à l'étape 50, la détermination d'une première information d'iso-luminance, en utilisant par exemple le procédé de la **Figure 4.** Typiquement, un couple de couleurs parmi les couples rouge-vert, rouge-bleu et vert-bleu de l'espace colorimétrique rouge-vert-bleu est considéré. L'étape 50 permet d'obtenir une valeur déterminée pour une première couleur (par exemple le vert) lorsque la deuxième couleur (par exemple le rouge) prend une valeur de référence par exemple $r_{ref}$=140 (correspondant à Rouge(140,0,0)). La valeur déterminée, par exemple $g_2$=120 pour la couleur (0,120,0) dans l'exemple plus haut, constitue ainsi la première information d'iso-luminance.

A l'étape optionnelle 51, une deuxième information d'iso-luminance peut être obtenue en utilisant également le procédé de la **Figure 4** par exemple. Cette deuxième information peut être obtenue pour un autre couple de couleurs, par exemple le rouge-bleu dans un exemple repris plus haut pour lequel la valeur Bleu=(0,0,170) est obtenue pour le rouge de référence (140,0,0).

Ainsi, deux informations d'iso-luminance chromatique, par exemple $g_2$=120 et $b_2$=170, sont obtenues pour deux couples différents de couleurs parmi les couples rouge-vert, rouge-bleu et vert-bleu de l'espace colorimétrique rouge-vert-bleu. Chaque information chromatique d'iso-luminance comprend une valeur déterminée, $g_2$=120 et $b_2$=170, pour une première couleur (vert et bleu respectivement) lorsque la deuxième couleur (rouge dans les deux cas) prend une valeur de référence $r_{ref}$=140 pour Rouge(140,0,0).

Dans cet exemple, les couleurs utilisées sont préférentiellement pures.

A l'étape 52, on calcule un différentiel $\delta_1$ de valeur la première valeur $g_2$ obtenue et une valeur par défaut de calibration correspondante (ici de la composante verte). En effet, l'idée ici est de corriger la calibration par défaut de l'écran 10.

On obtient donc les paramètres de calibration initiale de l'écran d'affichage 10. Ces paramètres peuvent être en mémoire du système 30 ou être fournis par un contrôleur (non représenté) dans l'écran 10. Ces paramètres permettent d'obtenir une valeur $g_{default}$ par défaut d'iso-luminance chromatique de la première couleur, ici verte, pour l'écran 10 lorsque la deuxième couleur, ici rouge, prend la valeur de référence $r_{ref}$=140 pour Rouge(140,0,0). Pour simplifier les explications, considérons que la calibration initiale d'iso-luminance de l'écran 10 est (140,140,140), signifiant que toutes les composantes couleur sont isoluminantes.

L'étape 52 consiste alors à calculer $\delta_1 = g_2 - g_{default}$.

Dans l'exemple $\delta_1$ = 120 - 140 = -20.

A noter que si l'étape 50 ne fournit pas une valeur d'iso-luminance chromatique à la valeur de référence (ici du rouge) considérée dans les paramètres de calibrage, un ajustement peut être prévu (par addition/soustraction par exemple d'un même nombre d'unités pour les deux couleurs de la configuration d'iso-luminance déterminée, de sorte que la deuxième couleur prenne la valeur de référence).

L'étape optionnelle 53 est similaire, pour le calcul d'un différentiel $\delta_2$ de valeur la deuxième valeur $b_2$ obtenue et une valeur par défaut de calibration correspondante (ici de la composante bleu).

Dans l'exemple $\delta_2 = b_2 - b_{default} = 170 - 140 = +30$.

A noter que la configuration d'iso-luminance pour le troisième couple de couleurs non traité peut être déterminée directement des deux configurations obtenues aux étapes 50 et 51. Dans notre exemple, la configuration d'iso-luminance vert-bleu doit représenter par exemple la valeur $b_2$ d'iso-luminance du bleu perçue par l'utilisateur lorsque le vert est mis à la valeur de référence 140. Les configurations montrent qu'à luminance constante, le vert est de 20 unités plus faible que le rouge, lui-même plus faire de 30 unités que le bleu. Aussi, la valeur $b_2$ d'iso-luminance est de 190 pour un vert de référence mis à (0,140,0).

Le procédé se poursuit à l'étape 54 par l'exploitation de ce ou ces différentiels pour ajuster la calibration de l'écran 10. Notamment, l'affichage d'un pixel (plus généralement de tous les pixels) est corrigé en fonction de la ou des informations d'iso-luminance chromatique déterminée $g_2$ et $b_2$, par exemple on ajuste le triplet de couleurs (généralement RGB) définissant le pixel selon une calibration initiale de l'écran d'affichage en fonction des deux différentiels $\delta_1$ et $\delta_2$.

Différentes réalisations de la correction de couleurs $(r_i,g_i,b_i)$ d'un pixel $p_i$ de l'écran peuvent être envisagées. Notons $(r_{new},g_{new},b_{new})$ les couleurs après correction.

Certaines réalisations opèrent à valeur constante d'une de ses composantes couleurs. Prenons l'exemple de la composante rouge $r_i$ inchangée (les mêmes explications s'appliquent pour les autres composantes) : $r_{new}=r_i$.

Dans un mode de réalisation, la correction du pixel comprend la modification d'une couleur du pixel d'une valeur égale audit différentiel de valeur. Par exemple, $g_{new}= g_i + \delta_1$ et $b_{new} = b_i + \delta_2$.

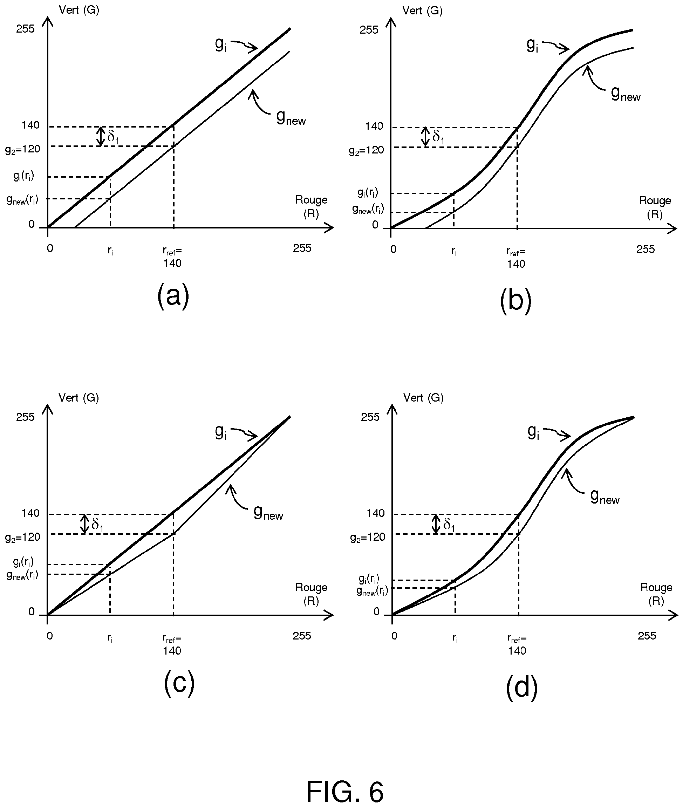

La **Figure 6a** illustre cette modification pour la composante verte $g_i$ dans une configuration d'écran où l'iso-luminance rouge-verte est linéaire. La **Figure 6b** illustre une modification similaire dans le cas où l'iso-luminance rouge-verte est quelconque.

Dans un autre mode de réalisation, la correction du pixel comprend la modification d'une couleur du pixel d'une valeur qui est fonction à la fois du différentiel de valeur et de la distance d'une autre couleur du pixel à la valeur de référence. L'idée ici est d'introduire une correction qui peut varier selon que l'on se situe plus ou moins loin de la valeur de référence à partir de laquelle les différentiels de correction $\delta_1$ et $\delta_2$ ont été déterminés. Elle permet notamment en définissant les valeurs extrêmes (0,0,0) et (255,255,255) comme étant invariantes par cette correction, de conserver des valeurs corrigées $r_{new},g_{new},b_{new}$ dans l'espace colorimétrique (donc entre 0 et 255 chacune).

Gardons le rouge comme valeur de référence. Les autres composantes verte et bleu sont corrigées de $\delta_1$ et $\delta_2$ respectivement lorsque la composante rouge vaut 140, et corrigées d'une valeur moindre (selon la distance de la composante rouge à 140) lorsque le pixel à afficher a une composante rouge différente de 140. La correction est nulle aux points 0 et 255. Des arrondis sont mis en place car les composantes couleurs sont nécessairement des valeurs entières.

Préférentiellement, la fonction modifiant $\delta_1$ et $\delta_2$ selon la distance de la composante rouge à 140 (donc la distance entre $r_{ref}$ et $r_i$) est préférentiellement linéaire par rapport à cette distance.

Cette configuration est illustrée par les **Figures 6c** (iso-luminance rouge-verte linéaire) et **6d** (iso-luminance rouge-verte non linéaire). Cette fonction est donc linéaire par morceaux de part et d'autre du point d'iso-luminance déterminé, (140, 160) ici pour le couple Rouge-Vert.

Dans ce cas, $g_{new}(r_i) = g_i(r_i) + \delta_1 * r_i / 140$ si $r_i<140$ et $g_{new}(r_i) = g_i(r_i) + \delta_1 * (255-r_i) / (255-140)$ si $r_i>140$.

Dans d'autres modes de réalisation, la correction du triplet de couleurs $(n_i,g_i,b_i)$ à afficher est effectué à luminance du pixel $p_i$ constante.

Prenons l'exemple suivant (d'autres formules existent pour l'homme de l'art) d'une luminance calculée à partir du triplet RGB de couleurs :

$$L(r_i,g_i,b_i) = 0{,}2126 * r_i + 0{,}7152 * g_i + 0{,}0722 * b_i$$

Ces autres modes de réalisation consistent à résoudre le système à trois inconnues formé des trois équations suivantes :

$$L(r_{new},g_{new},b_{new}) = L(r_i,g_i,b_i)$$

$$g_{new} = g_{new}(r_i)$$
$$b_{new} = b_{new}(r_i)$$

Le procédé de calibration d'un écran selon l'invention permet d'afficher des couleurs perçues de la même manière (relativement à leur iso-luminance) par différents utilisateurs utilisant différents écrans.

Les **Figures 7** et **8** illustrent l'application de la configuration d'iso-luminance à des fins sécuritaires, par exemple à la vérification d'identité (contrôle d'identité) ou au contrôle d'accès (voiture, coffre-fort, bâtiment, porte, base de données, service en ligne tel un compte en banque) reposant sur la sécurisation de systèmes informatiques, notamment par identification et/ou authentification.

La procédure d'identification ou authentification d'un individu peut être mise en œuvre par le système 1 de la **Figure 1.**

La **Figure 7** illustre des étapes préalables pour la mémorisation, dans le système 30, d'information d'identification/authentification de l'individu 2, par exemple un utilisateur du système informatique, aux fins de permettre une identification ou authentification ultérieure, selon les enseignements de l'invention.

A l'étape 70, un profil de l'utilisateur 2 est obtenu qui comporte un identifiant unique de l'utilisateur. Par exemple, l'utilisateur s'identifie auprès du système 30, par code PIN, par carte à puce, par login/mot de passe, par identification biométrique, ou par tout autre moyen.

A l'étape 71, une première information d'iso-luminance est déterminée pour un des yeux de l'utilisateur, en utilisant par exemple le procédé de la **Figure 4.** Typiquement, un couple de couleurs parmi les couples rouge-vert, rouge-bleu et vert-bleu de l'espace colorimétrique rouge-vert-bleu est considéré. L'étape 71 permet d'obtenir une valeur déterminée pour une première couleur (par exemple le vert) lorsque la deuxième couleur (par exemple le rouge) prend une valeur de référence par exemple (140,0,0). La valeur déterminée est par exemple $g_2=120$ comme dans l'exemple plus haut de la **Figure 5,** et constitue ainsi la première information d'iso-luminance déterminée.

A l'étape optionnelle 72, une deuxième information d'iso-luminance peut être obtenue pour le même œil, en utilisant également le procédé de la **Figure 4** par exemple. Cette deuxième information peut être obtenue pour un autre couple de couleurs, par exemple le rouge-bleu dans un exemple repris plus haut pour lequel la valeur Bleu=(0,0,170) est obtenue pour le rouge de référence $r_{ref}=140$ pour Rouge(140,0,0).

Ainsi, deux informations d'iso-luminance chromatique, par exemple $g_2=120$ et $b_2=170$, sont obtenues pour deux couples différents de couleurs parmi les couples rouge-vert, rouge-bleu et vert-bleu de l'espace colorimétrique rouge-vert-bleu. Chaque information chromatique d'iso-luminance comprend une valeur déterminée, $g_2=120$ et $b_2=170$, pour une première couleur (vert et bleu respectivement) lorsque la deuxième couleur (rouge dans les deux cas) prend une valeur de référence (140,0,0).

A l'étape 73, les deux informations d'iso-luminance chromatique, ici $g_2=120$ et $b_2=170$, pour l'œil considéré sont mémorisées en mémoire du système informatique 30 en association avec un identifiant de l'utilisateur (ici dans le profil de l'utilisateur).

Ces deux informations sont des informations d'iso-luminance chromatique de référence, notées $g_{ref}$ et $b_{ref}$, au sens où elles vont être utilisées dans le processus de la **Figure 8** pour valider ou non une identification ou authentification ultérieure de l'utilisateur 2. Elles sont de préférence stockées sous forme cryptée, et/ou dans une mémoire protégée (par exemple un module sécurisé).

Le procédé préalable de la **Figure 7** est ainsi terminé.

Comme illustré par la **Figure 8,** l'utilisateur 2 souhaite maintenant accéder à un service/système géré par le système 30 et nécessitant son identification ou authentification.

A l'étape 81, l'utilisateur 2 effectue une étape préliminaire d'identification. Cette étape vise notamment à récupérer le profil utilisateur et la ou les informations d'iso-luminance chromatique de référence $g_{ref}$ et $b_{ref}$ associées à l'utilisateur, avant la suite de la procédure d'identification/authentification (étapes 82 et suivantes).

De façon similaire à l'étape 70 ci-dessus, cette étape préliminaire d'identification peut se baser sur un code PIN fourni par l'utilisateur, une carte à puce propre à celui-ci, un login/mot de passe de l'utilisateur, une identification biométrique de l'utilisateur (morphométrie, empreinte digitale, image de l'iris), ou tout autre moyen.

Le procédé se poursuit par les étapes 82 et 83 permettant de déterminer la ou les informations d'iso-luminance chromatique $g_2$ et $b_2$ de l'œil pertinent de l'utilisateur pour la même couleur rouge de référence (140,0,0) que celle utilisée dans la **Figure 7.** Ces étapes sont similaires aux étapes 50 et 51 ou 71 et 72 décrites plus haut. Elles font donc appel au procédé de la **Figure 4** par exemple.

Dans un mode de réalisation visant à réduire les traitements de ces étapes, une étape 84 est prévue de détermination, en fonction de l'identification de l'utilisateur obtenue à l'étape 80, d'au moins une plage restreinte (par rapport à une plage de valeurs possibles) de valeurs de modification pour modifier dans le temps la couleur à modifier du motif multicolore, typiquement bicolore lors de la détermination ou des déterminations 82 et 83. Typiquement, le profil de modification 410 à utiliser pendant ces étapes 82 et 83 peut être une partie seulement de celui illustré en **Figure 3,** par exemple uniquement entre 80 et 160 (si l'on sait que l'utilisateur identifié a une valeur d'iso-luminance de référence d'environ 120). Cette plage restreinte peut être indiquée (via un identifiant) dans le profil utilisateur, ou être générée à la volée de façon aléatoire (autour de la valeur d'iso-luminance de référence de l'utilisateur).

Le procédé se poursuit à l'étape 85 par la comparaison de la ou des informations d'iso-luminance chromatique déterminée aux étapes 82 et 83 avec la ou les informations d'iso-luminance chromatique de référence correspondantes, telles que mémorisée à l'étape 73 pour l'utilisateur.

La comparaison n'est pas nécessairement stricte et peut notamment tenir compte d'une marge d'erreur, par exemple quelques unités sur l'échelle 0-255 des valeurs possibles de chaque composante couleur (étape 86).

L'identification ou authentification de l'utilisateur 2 n'est validée (étape 87) que si les informations d'iso-luminance correspondent, c'est-à-dire si $g_2$ et $b_2$ déterminées aux étapes 82 et 83 valent $g_{ref}$ et $b_{ref}$ respectivement, pour l'œil considéré. Dans ce cas, l'accès au service ou système est autorisé.

Autrement, l'identification ou authentification est refusée (étape 88).

Compte tenu du nombre discret et faible de configurations d'iso-luminance possible par rapport au nombre d'humains, l'identification ou authentification basée sur les informations d'iso-luminance selon l'invention est préférablement combinée à une identification/authentification forte de l'utilisateur (lors de l'étape 80 ci-dessus par exemple), par exemple à partir de données biométriques. Ainsi, l'identification ou authentification basée sur les informations d'iso-luminance selon l'invention sécurisé l'identification/authentification biométrique en ce qu'elle garantit que l'utilisateur est bien vivant.

L'exemple ci-dessus d'identification/authentification est basé sur la configuration d'iso-luminance perçue par un œil de l'individu. Cependant, il a été constaté que les deux yeux d'un individu ne perçoivent généralement pas les couleurs de la même manière, la différence de perception pouvant être légère ou significative. Il peut donc être prévu, dans un mode de réalisation particulier, d'identifier/authentifier l'individu en s'appuyant sur les configurations d'iso-luminance (décrites ci-dessus) des deux yeux. Par exemple, on peut effectuer une première étape de validation avec le premier œil (par exemple œil droit), puis une deuxième étape de validation avec l'autre œil. La sécurité de ce mode de réalisation est ainsi renforcée.

En variante, la différence de réponse entre les deux pupilles de l'individu peut être prise en compte et servir d'élément d'identification/authentification de l'individu aux fins sécuritaires susmentionnées. Par exemple, la différence entre les deux configurations d'iso-luminance (par exemple la différence de valeurs du vert lorsque le rouge est fixé à une valeur de référence).

Les exemples qui précèdent ne sont que des modes de réalisation de l'invention qui ne s'y limite pas.

**Revendications**

1. Procédé de contrôle personnalisé d'un processus dans un système informatique (1, 30), comprenant les étapes suivantes :

   déterminer (50, 51, 82, 83) au moins une information d'iso-luminance chromatique ($g_2$, $b_2$) pour un individu (2), la détermination d'une information d'iso-luminance comprenant les étapes suivantes :

   soumettre (41) l'individu à au moins un stimulus multicolore, typiquement bicolore, dynamique comprenant l'affichage, sur un périphérique d'affichage (10) du système informatique, d'un motif multicolore, typiquement bicolore, dont au moins deux couleurs sont inversées périodiquement à une fréquence dite de marquage ($F_{tag}$),
   contrôler (41) une modification dans le temps d'au moins une ($C_2$) des couleurs du motif multicolore lors de l'affichage du stimulus multicolore dynamique, pour faire varier la luminance affichée de cette couleur et faire varier une différence de luminance entre les deux couleurs,
   acquérir, (42, 43, 44) par un dispositif d'acquisition d'images (20), une réponse oscillatoire ($SIG_{resp}$) d'au moins une pupille de l'individu, lors de l'affichage du stimulus multicolore dynamique,
   générer (46, 48), à partir de la réponse acquise, un signal ($SIG_{power}$, $APPROX_{power}$) représentatif de la puissance de la réponse oscillatoire ($SIG_{resp}$) de la pupille en fonction de la modification dans le temps d'au moins une des deux couleurs et d'une différence de luminance entre les deux couleurs lors de l'affichage du stimulus multicolore dynamique, et
   déterminer (49) la configuration bicolore d'iso-luminance des deux couleurs dans le stimulus multicolore dynamique correspondant à un minimum (MIN) du signal représentatif de la puissance ($SIG_{power}$, $APPROX_{power}$), et

   utiliser (54, 85) l'au moins une information d'iso-luminance chromatique ainsi déterminée comme donnée d'entrée du processus dans le système informatique.

2. Procédé selon la revendication 1 pour la calibration d'un écran d'affichage (10) du système informatique, dans lequel l'affichage d'un pixel ($p_i$) est corrigé en fonction de l'au moins une information d'iso-luminance chromatique déterminée.

**3.** Procédé selon la revendication 2, dans lequel l'au moins une information d'iso-luminance chromatique ($g_2$, $b_2$) comprend, pour au moins un couple de couleurs parmi les couples rouge-vert, rouge-bleu et vert-bleu de l'espace colorimétrique rouge-vert-bleu, une valeur déterminée pour une première couleur ($C_2$) lorsque la deuxième couleur ($C_1$) prend une valeur de référence.

**4.** Procédé selon la revendication 3, dans lequel on obtient, pour une calibration initiale de l'écran d'affichage, une valeur par défaut d'iso-luminance chromatique ($g_{ref}$, $b_{ref}$) de la première couleur ($C_2$) lorsque la deuxième couleur ($C_1$) prend la valeur de référence ($r_{ref}$), et
la correction (54) d'un pixel ($p_i$) comprend l'ajustement de la première ou deuxième ou des deux couleurs du pixel en fonction du différentiel de valeur ($\delta_1$, $\delta_2$) entre la valeur par défaut d'iso-luminance chromatique ($g_{ref}$, $b_{ref}$) et la valeur déterminée d'iso-luminance chromatique ($g_2$, $b_2$).

**5.** Procédé selon la revendication 4, dans lequel la correction (54) du pixel ($p_i$) comprend la modification de la première ou deuxième couleur du pixel d'une valeur égale audit différentiel de valeur ($\delta_1$, $\delta_2$).

**6.** Procédé selon la revendication 4, dans lequel la correction (54) du pixel ($p_i$) comprend la modification de la première couleur du pixel d'une valeur qui est fonction à la fois du différentiel de valeur ($\delta_1$, $\delta_2$) et de la distance de la deuxième couleur ($r_i$) du pixel à la valeur de référence ($r_{ref}$).

**7.** Procédé selon la revendication 6, dans lequel cette fonction est linéaire par rapport à ladite distance entre la deuxième couleur du pixel et la valeur de référence.

**8.** Procédé selon la revendication 2 ou 3, comprenant la détermination de deux informations d'iso-luminance chromatique pour deux couples différents de couleurs parmi les couples rouge-vert, rouge-bleu et vert-bleu de l'espace colorimétrique rouge-vert-bleu, chaque information chromatique d'iso-luminance comprenant une valeur déterminée ($g_2$, $b_2$) pour une première couleur ($C_2$) lorsque la deuxième couleur ($C_1$) prend une valeur de référence ($r_{ref}$).

**9.** Procédé selon la revendication 8, dans lequel la correction (54) d'un pixel ($p_i$) sur l'écran d'affichage (10) comprend l'ajustement d'un triplet de couleurs définissant le pixel selon une calibration initiale de l'écran d'affichage, en fonction de deux différentiels de valeur ($\delta_1$, $\delta_2$) obtenus pour les deux couples de couleurs, chaque différentiel de valeur représentant la différence entre une valeur par défaut d'iso-luminance chromatique pour la première couleur du couple de couleurs lorsque la deuxième couleur prend la valeur de référence et la valeur déterminée d'iso-luminance chromatique.

**10.** Procédé selon la revendication 9, dans lequel l'ajustement du triplet de couleurs est effectué à luminance du pixel constante.

**11.** Procédé selon la revendication 9, dans lequel l'ajustement du triplet de couleurs est effectué à valeur constante d'une de ses composantes couleurs.

**12.** Procédé selon la revendication 1, dans lequel le processus utilisant l'au moins une information d'iso-luminance chromatique ($g_2$, $b_2$) est une procédure d'identification ou authentification de l'individu (2).

**13.** Procédé selon la revendication 12, comprenant en outre la comparaison (85, 86) de l'au moins une information d'iso-luminance chromatique déterminée ($g_2$, $b_2$) avec au moins une information d'iso-luminance chromatique de référence correspondante ($g_{ref}$, $b_{ref}$), mémorisée en mémoire d'un système informatique en association avec un identifiant de l'individu (2), et l'identification ou authentification de l'individu n'est validée (87) que si les informations d'iso-luminance correspondent.

**14.** Procédé selon la revendication 13, comprenant la détermination de deux informations d'iso-luminance chromatique ($g_2$, $b_2$) pour deux couples différents de couleurs parmi les couples rouge-vert, rouge-bleu et vert-bleu de l'espace colorimétrique rouge-vert-bleu, chaque information chromatique d'iso-luminance comprenant une valeur déterminée ($g_2$, $b_2$) pour une première couleur ($C_2$) lorsque la deuxième couleur ($C_1$) prend une valeur de référence ($r_{ref}$).

**15.** Procédé selon la revendication 13 ou 14, comprenant en outre une étape préliminaire (80) d'identification de l'individu (2) pour récupérer l'au moins une information d'iso-luminance chromatique de référence ($g_{ref}$, $b_{ref}$) associée à l'individu, avant ladite comparaison (85, 86).

**16.** Procédé selon l'une des revendications 12 à 15, comprenant en outre la détermination (84), en fonction de l'identification de l'individu ainsi obtenue, d'au moins une plage restreinte de valeurs de modification pour modifier dans le temps la couleur à modifier du motif multicolore, typiquement bicolore, lors de la détermination (82, 83) d'une information d'iso-luminance chromatique ($g_2$, $b_2$).

**17.** Système (1) de contrôle personnalisé d'un processus dans un système informatique, comprenant :

un sous-système (30) de détermination d'au moins une information d'iso-luminance chromatique ($g_2$, $b_2$) pour un individu (2), et
un module de contrôle de processus configuré pour utiliser l'au moins une information d'iso-luminance chromatique ainsi déterminée comme donnée d'entrée du processus dans le système informatique,
le sous-système de détermination d'une information d'iso-luminance comprenant :

un périphérique d'affichage (10),
un système informatique (31) de stimulation de l'individu par stimulus multicolore, typiquement bicolore, dynamique, le système informatique commandant l'affichage, sur le périphérique d'affichage, d'un motif multicolore, typiquement bicolore dont au moins deux couleurs sont inversées périodiquement à une fréquence dite de marquage ($F_{tag}$),
un contrôleur de couleurs (32) configuré pour modifier dans le temps au moins une ($C_2$) des deux couleurs du motif multicolore lors de l'affichage du stimulus multicolore dynamique, pour faire varier la luminance affichée de cette couleur et faire varier une différence de luminance entre les deux couleurs,
un dispositif d'acquisition d'images (20) pour acquérir une réponse oscillatoire ($SIG_{resp}$) d'au moins une pupille de l'individu, lors de l'affichage du stimulus multicolore dynamique,
un générateur d'indicateur ou biomarqueur (33) configuré pour générer, à partir de la réponse acquise, un signal ($SIG_{power}$, $APPROX_{power}$) représentatif de la puissance de la réponse oscillatoire ($SIG_{resp}$) de la pupille en fonction de la modification dans le temps d'au moins une des deux couleurs et d'une différence de luminance entre les deux couleurs lors de l'affichage du stimulus multicolore dynamique, et
une unité de détermination d'iso-luminance configurée pour déterminer la configuration d'iso-luminance des deux couleurs dans le stimulus multicolore dynamique correspondant à un minimum (MIN) du signal représentatif de la puissance ($SIG_{power}$, $APPROX_{power}$).

**Patentansprüche**

**1.** Personalisiertes Verfahren zur Steuerung eines Prozesses in einem Computersystem (1, 30) mit den folgenden Schritten:
Bestimmen (50, 51, 82, 83) mindestens einer chromatischen Isoluminanzinformation ($g_2$, $b_2$) für ein Individuum (2), wobei das Bestimmen einer Isoluminanzinformation die folgenden Schritte aufweist:

Aussetzen (41) des Individuums mindestens einem dynamischen mehrfarbigen, typischerweise zweifarbigen, Stimulus, welches das Anzeigen eines mehrfarbigen, typischerweise zweifarbigen Motivs auf einem Anzeigegerät (10) des Computersystems aufweist, wobei mindestens zwei Farben periodisch mit einer sogenannten Markierungsfrequenz ($F_{tag}$) invertiert werden,
Steuern (41) einer zeitlichen Änderung mindestens einer ($C_2$) der Farben des mehrfarbigen Motivs bei der Anzeige des dynamischen mehrfarbigen Stimulus, um die angezeigte Luminanz dieser Farbe zu variieren und einen Luminanzunterschied zwischen den beiden Farben zu variieren,
durch eine Bilderfassungsvorrichtung (20) erfolgendes Erfassen (42, 43, 44) einer Schwingungsantwort ($SIG_{resp}$) mindestens einer Pupille des Individuums bei der Anzeige des dynamischen mehrfarbigen Stimulus,
Erzeugen (46, 48), aus der erfassten Reaktion, eines Signals ($SIG_{power}$, $APPROX_{power}$), das die Stärke der Schwingungsantwort ($SIG_{resp}$) der Pupille in Abhängigkeit von der zeitlichen Veränderung mindestens einer der beiden Farben und einem Luminanzunterschied zwischen den beiden Farben beim Anzeigen des dynamischen mehrfarbigen Stimulus wiedergibt, und
Bestimmen (49) der zweifarbigen Konfiguration der Isoluminanz der beiden Farben in dem dynamischen mehrfarbigen Stimulus entsprechend einem Minimum (MIN) des die Stärke wiedergebenden Signals ($SIG_{power}$, $APPROX_{power}$),
Verwenden (54, 58) der so bestimmten mindestens einen chromatischen Isoluminanzinformation als Eingangsdaten des Prozesses in das Computersystem.

**2.** Verfahren nach Anspruch 1, zur Kalibrierung eines Anzeigebildschirms (10) des Computersystems, wobei die Anzeige eines Pixels ($p_i$) in Abhängigkeit von der bestimmten mindestens einen chromatischen Isoluminanzinformation korrigiert wird.

**3.** Verfahren nach Anspruch 2, bei welchem die mindestens eine chromatische Isoluminanzinformation ($g_2$, $b_2$) für mindestens ein Farbenpaar unter den Farbenpaaren rot-gelb, rot-blau und grün-blau des Rot-Grün-Blau-Farbraums einen Wert aufweist, der für eine erste Farbe ($C_2$) bestimmt wird, wenn die zweite Farbe ($C_1$) einen Referenzwert annimmt.

**4.** Verfahren nach Anspruch 3, bei welchem für eine anfängliche Kalibrierung des Anzeigebildschirms ein Standardwert der chromatischen Isoluminanz ($g_{ref}$, bref) der ersten Farbe ($C_2$) erhalten wird, wenn die zweite Farbe ($C_1$) den Referenzwert ($r_{ref}$) annimmt, und
das Korrigieren (54) eines Pixels ($p_i$) das Einstellen der ersten oder der zweiten oder der beiden Farben des Pixels in Abhängigkeit der Wertedifferenz ($\delta_1$, $\delta_2$) zwischen dem Standardwert der chromatischen Isoluminanz ($g_{ref}$, bref) und dem bestimmten Wert der chromatischen Isoluminanz ($g_2$, $b_2$) aufweist.

**5.** Verfahren nach Anspruch 4, bei welchem das Korrigieren (54) des Pixels ($p_i$) die Änderung der ersten oder der zweiten Farbe des Pixels um einen Wert gleich der Wertedifferenz ($\delta_1$, $\delta_2$) aufweist.

**6.** Verfahren nach Anspruch 4, bei welchem das Korrigieren (54) des Pixels ($p_i$) die Änderung der ersten Farbe um einen Wert aufweist, der gleichzeitig von der Wertedifferenz ($\delta_1$, $\delta_2$) und der Entfernung der zweiten Farbe ($r_i$) des Pixels von dem Referenzwert ($r_{ref}$) abhängt.

**7.** Verfahren nach Anspruch 6, bei welchem diese Funktion in Bezug auf die Entfernung der zweiten Farbe des Pixels von dem Referenzwert linear ist.

**8.** Verfahren nach Anspruch 2 oder 3, aufweisend das Bestimmen von zwei chromatischen Isoluminanzinformationen für zwei verschiedene Farbenpaare unter den Paaren rot-gelb, rot-blau und grün-blau des Rot-Grün-Blau-Farbraums, wobei jede chromatische Isoluminanzinformation einen bestimmten Wert ($g_2$, $b_2$) für eine erste Farbe ($C_2$) aufweist, wenn die zweite Farbe ($C_1$) einen Referenzwert ($r_{ref}$) annimmt.

**9.** Verfahren nach Anspruch 8, bei welchem das Korrigieren (54) eines Pixels ($p_i$) auf dem Anzeigebildschirm (10) das Einstellen eines Farbentriplets, welches das Pixel entsprechend einer anfänglichen Kalibrierung des Anzeigebildschirms definiert, in Abhängigkeit von zwei für die beiden Farbenpaare erhaltenen Wertedifferenzen ($\delta_1$, $\delta_2$) aufweist, wobei jede Wertedifferenz die Differenz zwischen einem Standardwert der chromatischen Isoluminanz für die erste Farbe des Farbenpaares, wenn die zweite Farbe den Referenzwert annimmt, und dem bestimmten Wert der chromatischen Isoluminanz angibt.

**10.** Verfahren nach Anspruch 9, bei welchem das Einstellen des Farbentripletts bei konstanter Pixelluminanz erfolgt.

**11.** Verfahren nach Anspruch 9, bei welchem das Einstellen des Farbentripletts bei einem konstanten Wert einer der Farbkomponenten desselben erfolgt.

**12.** Verfahren nach Anspruch 1, bei welchem der die mindestens eine chromatische Isoluminanzinformation ($g_2$, $b_2$) verwendende Prozess ein Vorgang zur Identifizierung oder Authentifizierung des Individuums (2) ist.

**13.** Verfahren nach Anspruch 12, ferner aufweisend den Vergleich (85, 86) der mindestens einen bestimmten chromatischen Isoluminanzinformation ($g_2$, $b_2$) mit mindestens einer entsprechenden chromatischen Isoluminanz-Referenzinformation ($g_{ref}$, $b_{ref}$), der in einem Speicher eines Computersystems in Verbindung mit einer Kennung des Individuums (2) gespeichert ist, und wobei die Identifizierung oder Authentifizierung des Individuums nur bestätigt (87) wird, wenn die Isoluminanzinformationen übereinstimmen.

**14.** Verfahren nach Anspruch 13, aufweisend das Bestimmen zweier chromatischer Isoluminanzinformationen ($g_2$, $b_2$) für zwei verschiedene Farbenpaare unter Paaren rot-gelb, rot-blau und grün-blau des Rot-Grün-Blau-Farbraums, wobei jede chromatische Isoluminanzinformation einen bestimmten Wert ($g_2$, $b_2$) für eine erste Farbe ($C_2$) aufweist, wenn die zweite Farbe ($C_1$) einen Referenzwert ($r_{ref}$) annimmt.

**15.** Verfahren nach Anspruch 13 oder 14, ferner aufweisend einen vorläufigen Schritt (80) des Identifizierens des Individuums (2), um die mindestens eine, mit dem Individuum verbundene chromatische Isoluminanz-Referenzinformation ($g_{ref}$, $b_{ref}$) vor dem Vergleich (85, 86) abzurufen.

16. Verfahren nach einem der Ansprüche 12 bis 15, ferner aufweisend das in Abhängigkeit der so erhaltenen Identifizierung des Individuums (2) erfolgende Bestimmen (84) eines begrenzten Werteänderungsbereichs zur zeitlichen Änderung der zu ändernden Farbe des mehrfarbigen, typischerweise zweifarbigen Motivs beim Bestimmen (82, 83) einer chromatischen Isoluminanzinformation ($g_2$, $b_2$).

17. Personalisiertes Steuersystem (1) für einen Prozess in einem Computersystem. welches aufweist:

ein Untersystem (30) zur Bestimmung mindestens einer chromatischen Isoluminanzinformation ($g_2$, $b_2$) für ein Individuum (2), und
ein Prozesssteuermodul, das dazu ausgebildet ist, die so bestimmte mindestens eine chromatischen Isoluminanzinformation als Eingabedaten des Prozesses in das Computersystem zu verwenden,
wobei das Untersystem zur Bestimmung mindestens einer chromatischen Isoluminanzinformation aufweist:

ein Anzeigegerät (10),
ein Computersystem (31) zur Stimulation des Individuums durch einen mehrfarbigen, typischerweise zweifarbigen Stimulus, wobei das Computersystem die Anzeige eines mehrfarbigen, typischerweise zweifarbigen Motivs auf dem Anzeigegerät steuert, wobei mindestens zwei Farben desselben periodisch mit einer sogenannten Markierungsfrequenz ($F_{tag}$) invertiert werden,
eine Farbsteuerung (32), die dazu ausgebildet ist, mindestens eine ($C_2$) der Farben des mehrfarbigen Motivs bei der Anzeige des dynamischen mehrfarbigen Stimulus zeitlich zu verändern, um die angezeigte Luminanz dieser Farbe zu variieren und einen Luminanzunterschied zwischen den beiden Farben zu variieren,
eine Bilderfassungsvorrichtung (20) zum Erfassen einer Schwingungsantwort ($SIG_{resp}$) mindestens einer Pupille des Individuums bei der Anzeige des dynamischen mehrfarbigen Stimulus,
einen Indikator- oder Biomarkergenerator (33), der dazu ausgebildet ist, aus der erfassten Reaktion ein Signals ($SIG_{power}$, $APPROX_{power}$) zu erzeugen, das die Stärke der Schwingungsantwort ($SIG_{resp}$) der Pupille in Abhängigkeit von der zeitlichen Veränderung mindestens einer der beiden Farben und einem Luminanzunterschied zwischen den beiden Farben beim Anzeigen des dynamischen mehrfarbigen Stimulus wiedergibt, und
eine Isoluminanz-Bestimmungseinheit, die dazu ausgebildet ist, die Isoluminanzkonfiguration der beiden Farben in dem dynamischen mehrfarbigen Stimulus entsprechend einem Minimum (MIN) des die Stärke wiedergebenden Signals ($SIG_{power}$, $APPROX_{power}$) zu bestimmen.

**Claims**

1. A method for controlling a computer process in a personalised manner in a computer system (1,30), comprising the following steps:

determining (50, 51, 82, 83) at least one chromatic iso-luminance information ($g_2$, $b_2$) for an individual (2), the determination of an iso-luminance information comprising the following steps:

submitting (41) the individual to at least one multicoloured, typically bicoloured, dynamic stimulus comprising displaying, on a display device (10) of the computer system, a multicoloured, typically bicoloured pattern, at least two colours of which are periodically inverted at a so-called marking frequency ($F_{tag}$),
controlling (41) a change overtime of at least one ($C_2$) of the colours of the multicolour pattern when displaying the dynamic multicolour stimulus, to vary the displayed luminance of that colour and to vary a luminance difference between the two colours,
acquiring, (42, 43, 44) by an image acquisition device (20), an oscillatory response ($SIG_{resp}$) of at least one pupil of the individual, when displaying the dynamic multicoloured stimulus,
generating (46, 48), from the acquired response, a signal ($SIG_{power}$, $APPROX_{power}$) representative of the power of the oscillatory response ($SIG_{resp}$) of the pupil as a function of the change over time of at least one of the two colours and of a difference in luminance between the two colours when displaying the dynamic multicolour stimulus, and
determining (49) the bicolour iso-luminance configuration of the two colours in the dynamic multicolour stimulus corresponding to a minimum (MIN) of the signal representative of the power ($SIG_{power}$, $APPROX_{power}$), and

using (54, 85) the at least one chromatic iso-luminance information thus determined as process input data in the computer system.

**2.** The method according to claim 1 for the calibration of a display screen (10) of the computer system, in which the display of a pixel ($p_i$) is corrected as a function of at least one determined chromatic iso-luminance information.

**3.** The method according to claim 2, wherein the at least one chromatic iso-luminance information ($g_2$, $b_2$) comprises, for at least one colour pair among the red-green, red-blue and green-blue pairs of the red-green-blue colour space, a value determined for a first colour ($C_2$) when the second colour ($C_1$) takes a reference value.

**4.** The method according to claim 3, in which a default chromatic iso-luminance value ($g_{ref}$, bref) of the first colour ($C_2$) is obtained for an initial calibration of the display screen when the second colour ($C_1$) takes the reference value (rref), and
the correction (54) of a pixel ($p_i$) comprises adjusting the first or second or both colours of the pixel according to the value differential ($\delta_1$, $\delta_2$) between the default value of chromatic iso-luminance ($g_{ref}$, $b_{ref}$) and the determined value of the chromatic iso-luminance ($g_2$, $b_2$).

**5.** The method according to claim 4, wherein the correction (54) of the pixel ($p_i$) comprises modifying the first or second colour of the pixel by a value equal to said value differential ($\delta_1$, $\delta_2$).

**6.** The method according to claim 4, wherein the correction (54) of the pixel ($p_i$) comprises modifying the first colour of the pixel by a value that is a function of both the value differential ($\delta_1$, $\delta_2$) and the distance of the second colour ($r_i$) of the pixel from the reference value (rref).

**7.** The method according to claim 6, wherein this function is linear with respect to said distance between the second colour of the pixel and the reference value.

**8.** The method according to claim 2 or 3, comprising determining two chromatic iso-luminance information for two different colour pairs among the red-green, red-blue and green-blue pairs of the red-green-blue colour space, each chromatic iso-luminance information comprising a determined value ($g_2$, $b_2$) for a first colour ($C_2$) when the second colour ($C_1$) takes a reference value (rref).

**9.** The method according to claim 8, wherein the correction (54) of a pixel ($p_i$) on the display screen (10) comprises adjusting a colour triplet defining the pixel according to an initial calibration of the display screen, based on two value differentials ($\delta_1$, $\delta_2$) obtained for the two colour pairs, each value differential representing the difference between a default value of chromatic iso-luminance for the first colour of the colour pair when the second colour takes the reference value and the determined value of chromatic iso-luminance.

**10.** The method according to claim 9, wherein the colour triplet adjustment is performed at constant pixel luminance.

**11.** The method according to claim 9, wherein the adjustment of the colour triplet is performed at constant value of one of its colour components.

**12.** The method according to claim 1, wherein the process using the at least one chromatic iso-luminance information ($g_2$, $b_2$) is a procedure for identifying or authenticating the individual (2).

**13.** The method according to claim 12, further comprising comparing (85, 86) the at least one determined chromatic iso-luminance information ($g_2$, $b_2$) with at least one corresponding reference chromatic iso-luminance information ($g_{ref}$, $b_{ref}$) stored in the memory of a computer system in association with an identifier of the individual (2), and validating (87) the identification or authentication of the individual only if the iso-luminance information matches.

**14.** The method according to claim 13, comprising determining two pieces of chromatic iso-luminance information ($g_2$, $b_2$) for two different colour pairs among the red-green, red-blue and green-blue pairs of the red-green-blue colour space, each chromatic iso-luminance information comprising a determined value ($g_2$, $b_2$) for a first colour ($C_2$) when the second colour ($C_1$) takes a reference value (rref).

15. The method according to claim 13 or 14, further comprising a preliminary step (80) of identifying the individual (2) to retrieve the at least one reference chromatic iso-luminance information ($g_{ref}$, $b_{ref}$) associated with said individual, prior to said comparison (85, 86).

16. The method according to one of claims 12 to 15, further comprising determining (84), based on the identification of the individual thus obtained, at least a limited range of change values for changing over time the colour to be changed of the multicoloured, typically bicolour, pattern when determining (82, 83) chromatic iso-luminance information ($g_2$, $b_2$).

17. A system (1) for controlling a computer process in a personalised manner in a computer system, comprising:

a subsystem (30) for determining at least one chromatic iso-luminance information ($g_2$, $b_2$) for an individual (2), and
a process control module configured to use the at least one chromatic iso-luminance information thus determined as process input data into the computer system,
the subsystem for determining iso-luminance information comprising:

a display device (10),
a computer system (31) for stimulating the individual by means of a multicoloured, typically bicoloured, dynamic stimulus, the computer system controlling the display, on the display device, of a multicoloured, typically bicoloured, pattern, at least two colours of which are periodically inverted at a so-called marking frequency ($F_{tag}$),
a colour controller (32) configured to change over time at least one ($C_2$) of the two colours of the multicolour pattern when displaying the dynamic multicolour stimulus, to vary the displayed luminance of that colour and to vary a luminance difference between the two colours,
an image acquisition device (20) for acquiring an oscillatory response ($SIG_{resp}$) of at least one pupil of the individual, when displaying the dynamic multicolour stimulus,
an indicator or biomarker generator (33) configured to generate, from the acquired response, a signal ($SIG_{power}$, $APPROX_{power}$) representative of the power of the oscillatory response ($SIG_{resp}$) of the pupil as a function of the change over time of at least one of the two colours and a difference in luminance between the two colours when displaying the dynamic multicolour stimulus, and
an iso-luminance determination unit configured to determine the iso-luminance configuration of the two colours in the dynamic multicolour stimulus corresponding to a minimum (MIN) of the signal representative of the power ($SIG_{power}$, $APPROX_{power}$).

EP 3 599 986 B1

FIG. 1

temps

FIG. 2

25

FIG. 3

FIG. 4

FIG. 5

FIG. 7

FIG. 8

FIG. 6

**RÉFÉRENCES CITÉES DANS LA DESCRIPTION**

**Documents brevets cités dans la description**

- US 5141305 A

**Littérature non-brevet citée dans la description**

- **CAVANAGH P. et al.** *Screening for color blindness using optokinetic nystagmus,* 1984

- **CHAUDHURI A. et al.** *A new technique for estimating chromatic isoluminance in humans and monkeys,* 1990